# EUROPEAN PATENT APPLICATION

(11) **EP 4 431 091 A1**
(43) Date of publication of application: **18.09.2024**
(21) Application number: 23382255.0
(22) Date of filing: 17.03.2023
(51) Int. Cl.: A61K 31/197, A61K 31/198, A61K 31/4045, A61K 36/185, A61K 36/66, A61K 36/84, A61P 25/20, A61P 43/00, A23L 33/105, A23L 33/15, A23L 33/175

(54) **COMPOSITION WITH GLYCINE FOR USE TREATING INSOMNIA DISORDER**

(71) Applicant: Elaborados Dietéticos S.A.U., 08754 El Papiol (ES)
(72) Inventor: NARRO, Andrea, 08754 EL PAPIOL (ES); RISCO, Ester, 08754 EL PAPIOL (ES); GRECO, Giuseppe, 08754 EL PAPIOL (ES); GAYA, Marc, 08754 EL PAPIOL (ES); GAYA, Carla, 08754 EL PAPIOL (ES)
(74) Representative: ZBM Patents - Zea, Barlocci & Markvardsen

(57) **Abstract**

A composition is provided comprising glycine in a range of between 5 and 500 mg, γ-aminobutyric acid (GABA), at least one vegetal extract, and melatonin; and uses and methods thereof for treating a sleep disorder, in particular insomnia disorder. Products comprising the composition, particularly food supplements, and their methods of production are also provided.

## Description

### FIELD OF THE INVENTION

The present invention generally relates to the field of nutraceuticals for sleep disorders, in particular insomnia.

### BACKGROUND ART

Sleep is a basic human need that contributes to optimal health and vitality and has a multifactorial effect on the body: it reduces energy consumption and increases the recovery of the energy storage in the brain, regulates the adaptive and innate immune response, and contributes to memory consolidation (the fixing of acquired information in the brain).

The sympathetic nervous system and the hypothalamic-pituitary-adrenal axis, two effector systems responsible for regulating the immune response (innate and adaptive), are both influenced by sleep. During sleep, there is a decrease in the release of cortisol, norepinephrine, and adrenaline, and an increase in hormones that affect cell growth, such as growth hormones, melatonin, and prolactin. Prolactin and growth hormone influence the differentiation and formation of new T cells and stimulate the function of type 1 cytokines that control the antigenic response of lymphocytes. When sleep is inadequate, the immune system produces a reduced number of antibodies, which are involved in the body's defensive reactions.

Good sleep is sleep that is satisfactory, i.e., sufficient in duration and of good quality. Unsatisfactory sleep that occurs continuously causes more serious effects, such as often feeling very tired, having uncontrolled emotions, difficulty concentrating, difficulty remembering or thinking clearly, unsatisfactory work, depression, and difficulty solving problems.

Sleep disorders are highly prevalent and have a significant impact on people's quality of life and daytime functioning. Poor sleep can severely affect daytime performance, both socially and at work, and increases the risk of occupational and traffic accidents, poor quality of life and poor overall health.

Maintaining an optimal quality of sleep is also very important for neural functioning. Sleep and insomnia influence the different connections of the brain. During sleep, there is a spontaneous fusion of the glia and the neurons by the synapses, which leads to the formation of cell networks. The properties of the network are altered by synapses and signal molecules. During sleep, old superfluous memories are erased, new ones are strengthened, and the neuromuscular cycles are strengthened.

According to the 5th edition of the Diagnostic and Statistical Manual of Mental Disorders criteria (insomnia DSM-5 criteria), insomnia disorder, previously called "primary insomnia", is a sleep disorder characterized by difficulty in sleep initiation, difficulty in sleep maintenance or early morning awakening, occurring at least 3 times per week for at least three months, with significant personal distress and daytime functional impairments.

Insomnia is increasingly recognized as a major public health concern. It is now recognized that sleep disturbances are associated with neurocognitive dysfunctions, attention deficits, impaired cognitive performance, depression, anxiety, stress, asthma, gastroesophageal reflux, hypertension, cardiovascular diseases, and type 2 diabetes.

Treatments for sleep problems are generally recommended for short-term use due to their limitations such as possible dependence and tolerance to long-term use. An ideal treatment for insomnia should contribute to improved sleep latency and sleep duration with limited awakenings without significant adverse effects such as daytime somnolence or decreased alertness.

*Buenas Noches* (ELADIET^{®}) is a food supplement for insomnia that contributes to reduce the time taken to fall sleep, to maintain a natural sleep and to improve the quality of natural sleep. It contains melatonin, GABA, valerian, *Californian poppy*, *Passiflora*, vitamin B3 and B6: melatonin contributes to the reduction of the time taken to fall asleep, valerian helps to maintain a natural sleep, *Californian poppy* contributes to optimal relaxation and improves the quality of natural sleep, and vitamins B3 and B6 contribute to the normal functioning of the nervous system.

### SUMMARY OF THE INVENTION

One problem to be solved by the present invention is to improve the sleeping quality in a subject suffering from a sleep disorder, particularly insomnia disorder, e.g., by reducing sleep latency or wakefulness, increasing total sleep time, actual sleep time, bedtime or sleep efficiency, or improving the insomnia severity or sleepiness.

The solution is based on the provision of a composition comprising glycine in small amounts, GABA, at least one vegetal extract selected from the group consisting of *Valerian* root dry extract, *Passiflora* dry extract, and *Californian poppy* dry extract, and melatonin. An embodiment of the composition of the invention can be also referred herein to as *Buenas Noches Total.*

In this scenario, inventors have determined in the working examples provided herein that the treatment with *Buenas Noches Total* is significantly more effective than *Buenas Noches,* with the only difference being that *Buenas Noches Total* contains glycine in small amounts, particularly between 25 and 150 mg.

Particularly, EXAMPLE 1 shows the clinical efficacy of *Buenas Noches* and *Buenas Noches Total* through the evaluation of insomnia severity (by Insomnia Severity Index, ISI) and sleepiness (by Epworth Sleepiness Scale, ESS). With *Buenas Noches Total* they obtained a ISI decline rate of 53% and an ESS decline ratio of 24%, significantly major than those obtained for *Buenas Noches.* Particularly, the improvement of ISI score was 60.6% greater for *Buenas Noches Total* group compared to *Buenas Noches* group (from 33% reduction to 53% reduction), and the improvement of ESS score was 380% greater for *Buenas Noches Total* group compared to *Buenas Noches* group (from 5% reduction to 24% reduction).

Further, EXAMPLE 2 demonstrates the clinical efficacy of *Buenas Noches* and *Buenas Noches Total* by Kronohealth monitoring. In particular, they assessed sleep latency, total sleep time, wake after sleep onset (WASO), actual sleep time and sleep efficiency. After 7 days of treatment, *Buenas Noches Total* reduced the sleep latency in 25 minutes (reduction of 74% in comparison with baseline), which was 32.14% greater than *Buenas Noches* group (from 56% reduction to 74% reduction).

Moreover, after 7 days of treatment, *Buenas Noches Total* increased the actual sleep time for 83 minutes compared to baseline, representing a 25.6% increase. Particularly, the actual sleep time was 34 minutes greater for *Buenas Noches Total* group compared to *Buenas Noches* group (from 49 min to 83 min), which represents a 71.8% increase (from 14.9% to 25.6%).

In addition, after 7 days of treatment, *Buenas Noches Total* group obtained a sleep efficiency of 90%, which represents a 11.1% increase compared to baseline (from 81% at baseline to 90% at day 7).

Therefore, inventors have demonstrated through different sleep-related parameters that *Buenas Noches Total* shows unexpected differential results on sleep quality compared to *Buenas Noches,* concluding that glycine, in combination with the other compounds, is the main compound responsible for these effects.

The evidence provided in these clinical studies makes it plausible that compositions with small amounts of glycine similar to that of the tested composition *Buenas Noches Total* could also be useful in improving the sleep quality in subjects suffering from insomnia. That is, although specific compositions were tested in the trials with specific amounts, the effects shown are plausible for other compositions such as those claimed. Further, results can also be extrapolated to sleep disorders other than insomnia.

Accordingly, a first aspect of the invention relates to a composition comprising glycine in a range of between 5 and 500 mg; γ-aminobutyric acid (GABA); at least one vegetal extract selected from the group consisting of *Valerian* root dry extract, *Passiflora* dry extract, and *Californian poppy* dry extract; and melatonin.

Another aspect of the present invention relates to a nutraceutical product, a food supplement, a medical food, a food product a pharmaceutical product, or a veterinary product, comprising an effective amount of any of the compositions disclosed herein together with appropriate amounts of acceptable excipients.

In another aspect, the present invention relates to the compositions described herein for use in treating sleep disorders, or alternatively, to a method of treating sleep disorders, the method comprising administering an effective amount of the composition to a subject in need thereof.

Other aspects of the present invention are related to other uses and methods described hereinafter, and also to methods of production of the compositions and products of the invention.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### DESCRIPTION OF DRAWINGS

**FIG. 1** shows the clinical trial schedule for primary insomnia where subjective sleep quality was evaluated by Insomnia Severity Index (ISI) and Epworth Sleepiness Scale (ESS) scores.
**FIG. 2** shows the mean of ISI and ESS scores obtained at baseline and after 1 week of treatment. *p<0.05; **p<0.01; ***p<0.001, in comparison with baseline (T0).
**FIG. 3** shows the reduction of insomnia and daytime sleepiness though ISI and ESS decline rate, obtained in different groups.
**FIG. 4** shows the clinical trial schedule for primary insomnia where objective sleep quality was evaluated by Kronohealth monitoring. A picture of a Kronohealth monitoring device is shown.
**FIG. 5** shows the sleep latency (mean) and actual sleep time (minutes of increase compared to baseline), obtained in the different treatment groups. T0 corresponds to the mean of data from the previous 7 days without treatment. ***p<0.001, compared to baseline (T0).

### DETAILED DESCRIPTION OF THE INVENTION

### Composition

An aspect of the present invention relates to a composition comprising glycine in a range of between 5 and 500 mg; γ-aminobutyric acid (GABA); at least one vegetal extract selected from the group consisting of *Valerian* root dry extract, *Passiflora* dry extract, and *Californian poppy* dry extract; and melatonin. In an embodiment, the composition comprises glycine in a range of between 5 and 500 mg; GABA in a range of between 5 and 500 mg, particularly between 25 and 250 mg; at least one vegetal extract selected from the group consisting of *Valerian* root dry extract, *Passiflora* dry extract, and *Californian poppy* dry extract; and melatonin in an amount of less than 10 mg.

Glycine is a neurotransmitter in the central nervous system, and in high amounts (doses ranging from 3 to 5 grams) has been shown to have a number of effects that can contribute to improved sleep quality. However, inventors have seen that small amounts of glycine unexpectedly improve the sleep quality, together with other active ingredients. Particularly, glycine included in the composition is in a range of between 5 and 500 mg, between 5 and 400 mg, between 5 and 300 mg, between 5 and 200 mg, between 5 and 100 mg, between 10 and 75 mg, between 15 and 60 mg, between 25 and 150 mg, between 25 and 100 mg, between 25 and 75 mg, or between 30 and 60 mg, and particularly between 25 and 150 mg. Glycine can be in an amount of less than: 500 mg, 400 mg, 300 mg, 200 mg, 100 mg, 90 mg, 80 mg, 75 mg, 70 mg, 65 mg, 60 mg, 55 mg, or 50 mg. More particularly, glycine is in an amount of about 5 mg, about 10 mg, about 15 mg, about 20 mg, about 25 mg, about 30 mg, about 35 mg, about 40 mg, about 45 mg, 50 mg, about 55 mg, about 60 mg, about 65 mg, or about 70 mg, and particularly of about 50 mg. Even more particularly, glycine is in an amount of 50 mg.

GABA is a naturally occurring neurotransmitter in the brain involved in various brain functions, including sleep. In some embodiments, the composition comprises GABA in a range of between 5 and 500 mg, between 10 and 400 mg, between 30 and 300 mg, between 50 and 250 mg, between 50 and 200 mg, or between 50 and 150 mg, and particularly between 50 and 250 mg. GABA can be in an amount of about 10 mg, about 25 mg, about 50 mg, about 75 mg, about 80 mg, about 90 mg, about 100 mg, about 110 mg, about 120 mg, about 130 mg, about 150 mg, about 200 mg, about 250 mg, about 300 mg, about 350 mg, about 400 mg, about 450 mg, or about 500 mg, and particularly about 100 mg. More particularly, GABA is in an amount of 100 mg.

The composition of the present invention also comprises at least one vegetal extract selected from the group consisting of valerian, *Passiflora,* and *Californian poppy* extracts.

Valerian extract is derived from the root of the valerian plant, i.e., valerian root dry extract (*Valerianae radix*), and helps to maintain a natural sleep. Valerian root dry extract can be made from the following species of valerian: *Valeriana officinalis* L., *Valeriana edulis*, *Valeriana jatamansi*, *Valeriana celtica*, *Valeriana wallichii*, or *Valeriana sitchensis.* In an embodiment, the valerian root dry extract is made of *Valeriana officinalis* L. In some embodiments, the composition comprises valerian extract in a range of between 20 and 200 mg, between 50 and 150, between 60 and 140 mg, between 70 and 130 mg, between 80 and 120 mg, or between 90 and 110 mg, and particularly between 80 and 120 mg. The valerian extract can be in an amount of about 50 mg, about 60 mg, about 70 mg, about 80 mg, about 90 mg, about 100 mg, about 110 mg, about 120 mg, about 130 mg, about 140 mg, about 150 mg, or about 200 mg, and particularly of about 100 mg. More particularly, the valerian extract is in an amount of 100 mg.

*Passiflora* dry extract (i.e., *Passiflora herba*) can be made from the following species of *Passiflora: Passiflora incarnata* (*Passiflora edulis*)*, Passiflora alata*, *Passiflora caerulea*, or *Passiflora quadrangularis.* In an embodiment, the *Passiflora* dry extract is made of *Passiflora incarnata* (*Passiflora edulis*). In some embodiments, the composition comprises *Passiflora* dry extract in a range of between 20 and 200 mg, between 30 and 150, between 40 and 140 mg, between 50 and 130 mg, between 60 and 120 mg, between 60 mg and 90 mg, or between 70 mg and 80 mg, and particularly between 60 and 90 mg. The *Passiflora* dry extract can be in an amount of about 50 mg, about 55 mg, about 60 mg, about 65 mg, about 70 mg, about 75 mg, about 80 mg, about 90 mg, about 100 mg, about 110 mg, about 120 mg, about 130 mg, about 140 mg, or about 150 mg, and particularly of about 75 mg. More particularly, the *Passiflora* dry extract is in an amount of 75 mg.

*Californian poppy* extract (i.e., *Eschscholziae herba*) contributes to optimal relaxation and improves the quality of natural sleep, and can be made from the following species of *Californian poppy*: *Eschscholzia californica*, *Eschscholzia lemmonii*, *Eschscholzia minutiflora*, *Eschscholzia ramosa*, or *Eschscholzia tenuifolia.* In an embodiment, the *Californian* poppy dry extract is made of *Eschscholzia californica.* In some embodiments, the composition comprises *Californian poppy* extract in a range of between 1 and 100 mg, between 5 and 75, between 5 and 50, between 10 and 40 mg, between 15 and 30 mg, or between 20 and 30 mg, and particularly between 15 and 30 mg. The *Californian poppy* extract can be in an amount of about 1 mg, about 5 mg, about 10 mg, about 15 mg, about 20 mg, about 25 mg, about 30 mg, about 35 mg, about 40 mg, or about 45 mg, and particularly of about 25 mg. More particularly, the *Californian poppy* extract is in an amount of 25 mg.

In an embodiment, the composition comprises *Valerian* root dry extract, *Passiflora* dry extract, and *Californian poppy* dry extract.

The composition of the present invention also comprises melatonin in an amount of less than 10 mg. Melatonin is a hormone that plays an important role in regulating the sleep-wake cycle. The body's production of melatonin is controlled by the circadian rhythm, which is the internal biological clock that regulates many of the body's physiological processes, including sleep and wakefulness. Melatonin not only helps to promote sleep onset but also helps to regulate the timing and quality of sleep. Particularly, melatonin included in the composition is in an amount of less than: 10 mg, 9 mg, 8 mg, 7 mg, 6 mg, 5 mg, 4 mg, 3 mg, 2 mg, or 1 mg, and particularly of less than 2 mg. In a more particular embodiment, melatonin is in an amount of less than 1.95 mg, 1.90 mg, 1.85 mg, or 1.80 mg. More particularly, melatonin is in an amount of about 1 mg, about 1.50 mg, about 1.60 mg, about 1.70 mg, about 1.75 mg, about 1.80 mg, about 1.85 mg, about 1.90 mg, about 1.95 mg, about 2 mg, or about 2.5 mg, and particularly of about 1.85 mg. Even more particularly, melatonin is in an amount of 1.85 mg.

Vitamins play an important role in maintaining overall health and can also contribute to promoting healthy sleep. In some embodiments, the composition further comprises at least one vitamin. Vitamins include, e.g., vitamin B, vitamin D, vitamin C or vitamin E. In an embodiment, vitamin is vitamin B. Particularly, vitamin B is selected from the group consisting of vitamin B3, vitamin B5 and vitamin B6. More particularly, vitamin B is vitamin B3 and/or vitamin B6. Non-limiting examples of vitamin B6 are: pyridoxine (PN), pyridoxal (PL), pyridoxamine (PM), pyridoxine hydrochloride (PN-HCI), pyridoxal-5-phosphate (PLP), and pyridoxamine-5-phosphate (PMP). Non-limiting examples of vitamin B3 are: niacin (nicotinic acid), niacinamide (nicotinamide), inositol hexanicotinate, nicotinamide riboside (NR), nicotinamide mononucleotide (NMN), and nicotinic acid adenine dinucleotide phosphate (NAADP). In an embodiment, vitamin B6 is pyridoxine hydrochloride. In another embodiment, vitamin B3 is nicotinamide. In an embodiment, vitamin is in a range of between 0.1 and 50 mg, between 0.5 and 40 mg, between 0.5 and 30 mg, or between 1 and 25 mg, and particularly between 1 and 20 mg.

Particularly, vitamin B3 is in a range of between 1 and 50 mg, between 5 and 30 mg, between 5 and 30 mg, between 10 and 20 mg, or between 12 and 18 mg, and particularly between 10 and 20 mg. More particularly, vitamin B3 is in an amount of about 1 mg, about 5 mg, about 10 mg, about 12.5 mg, about 14 mg, about 15 mg, about 16 mg, about 17 mg, about 18 mg, about 20 mg, about 25 mg, about 30 mg, about 40 mg, about 45 mg, or about 50 mg, and particularly of about 16 mg. Even more particularly, vitamin B3 is in an amount of 16 mg.

Particularly, vitamin B6 is in a range of between 0.1 mg and 5 mg, between 0.5 and 3 mg, between 0.8 and 2 mg, between 1 and 1.8 mg, or between 1.2 and 1.6 mg, and particularly between 1 and 1.8 mg. More particularly, vitamin B6 is in an amount of about 0.1 mg, about 0.5 mg, about 1 mg, about 1.1 mg, about 1.2 mg, about 1.3 mg, about 1.4 mg, about 1.5 mg, about 1.6 mg, about 1.7 mg, about 1.8 mg, about 1.9 mg, about 2 mg, about 2.5 mg, about 3 mg, about 4 mg, or about 5 mg, and particularly of about 1.4 mg. Even more particularly, vitamin B6 is in an amount of 1.4 mg.

In a particular embodiment, the composition comprises glycine in a range of between 25 and 150 mg; GABA in a range of between 50 and 250 mg; at least one vegetal extract selected from the group consisting of *Valerian* root dry extract, *Passiflora* dry extract, and *Californian poppy* dry extract; and melatonin in an amount of less than 2 mg.

In a particular embodiment, the composition comprises glycine in a range of between 25 and 150 mg; GABA in a range of between 50 and 250 mg; at least one vegetal extract selected from the group consisting of *Valerian* root dry extract, *Passiflora* dry extract, and *Californian poppy* dry extract; melatonin in an amount of less than 2 mg; and at least one vitamin in a range of between 0.1 and 50 mg, particularly between 1 and 20 mg.

In a particular embodiment, the composition comprises glycine in a range of between 25 and 150 mg; GABA in a range of between 50 and 250 mg; *Valerian* root dry extract in a range of between 80 and 120 mg; *Passiflora* dry extract in a range of between 60 and 90 mg; *Californian poppy* dry extract in a range of between 15 and 30 mg; melatonin in an amount of less than 2 mg; and at least one vitamin in a range of between 0.1 and 50 mg, particularly between 1 and 20 mg.

In a particular embodiment, the composition comprises glycine in a range of between 25 and 150 mg; GABA in a range of between 50 and 250 mg; *Valerian* root dry extract in a range of between 80 and 120 mg; *Passiflora* dry extract in a range of between 60 and 90 mg; *Californian poppy* dry extract in a range of between 15 and 30 mg; melatonin in an amount of less than 2 mg; vitamin B3 in a range of between 10 and 20 mg; and vitamin B6 in a range of between 1 and 1.8 mg.

In a more particular embodiment, the composition comprises 50 mg of glycine, 100 mg of GABA, 100 mg of *Valerian* root dry extract, 75 mg of *Passiflora* dry extract, 25 mg of *Californian poppy* dry extract, 16 mg of vitamin B3, 1.4 mg of vitamin B6, and 1.85 mg of melatonin. This particular embodiment corresponds to the active ingredients of the composition of study in working examples, i.e., *Buenas Noches Total.*

In an embodiment, the composition further comprises at least one additional amino acid besides glycine and GABA. Particularly, the composition comprises between one and three additional amino acids. In a particular embodiment, the additional amino acid is selected from the group consisting of threonine, tryptophan, theanine and glutamine.

In an embodiment, the composition does not comprise at least one ingredient selected from the group consisting of milk proteins (e.g., hydrolyzed casein peptides), *Bacopa monnieri* extract, and *Gingko Biloba* extract.

The active ingredients of a composition of the present invention can be formulated in a single product (i.e., as unit dose) or in different products that can be administered in combination. Similarly, the composition according to the invention can be formulated mixed with one or more other active ingredients. The active ingredients may be formulated in a single product, or in separate forms to be administered sequential or consecutively in any order, wherein the form comprising the composition of the present invention, is administered prior to, substantially simultaneously, after or in between the administration of the other active ingredient(s).

The unit dose of a product (e.g., a tablet) is typically expressed in terms of the amount of the active ingredient contained in the product. The amounts of the composition of the invention detailed herein refer to a unit dose. That is, when referring to 50 mg of glycine, reference is made to the product containing 50 mg of glycine in total, i.e., each dose contains 50 mg of glycine.

### Medical uses and clinical outcomes of the composition

As discussed herein, the composition of the present invention (e.g., *Buenas Noches Total)* presents a high efficacy in improving the sleeping quality in a subject suffering from insomnia disorder (EXAMPLES 1 and 2). The composition has shown a significant beneficial effect in reducing the insomnia severity (reduction of ISI score), reducing sleepiness (reduction of ESS score), reducing sleep latency, increasing actual sleep time and improving insomnia severity. Therefore, the experimental data included herein provides evidence that it is plausible that the composition would have significant positive effects in other sleep-related diseases or disorders.

Accordingly, the present invention provides compositions for use in treating sleep disorders. It is understood that the uses disclosed herein can alternatively be formulated as a method of treating sleep disorders, the method comprising administering an effective amount of the composition to the subject in need thereof.

Alternatively, the composition of the present invention is:
- for use in improving a sleep disorder;
- for use in promoting (or supporting) sleep;
- for use in sleep support;
- for use in improving sleep quality;
- for the treatment of sleep disorders and the induction of restorative sleep function; or
- for modifying or improving the sleep-wake cycle.

Said uses can alternatively be formulated as a method of: improving a sleep disorder; promoting (or supporting) sleep; supporting sleep; improving sleep quality, treating sleep disorders and inducing restorative sleep function; or modifying or improving the sleep-wake cycle, the method comprising administering an effective amount of the composition to a subject in need thereof.

The terms "treat", "treating", "treatment", "therapy", as used herein refers to a clinical intervention to reduce the seriousness or severity of the disease or condition (e.g., reduce the extent of insomnia disorder); ameliorate or eliminate one or more symptoms or sequelae associated with a disease or condition (e.g., insomnia disorder); prevent (e.g., suppress or inhibit) a disease/disorder or condition; cure the disease or condition; delay the onset of the disease or condition; or the provision of beneficial effects to a subject with a disease or condition, without necessarily curing the disease or condition.

In some embodiments, the term refers to a clinical intervention to improve one or more symptoms; improve one or more sequelae; prevent (e.g., suppress, inhibit or delay) one or more symptoms; prevent (e.g., suppress, inhibit or delay) one or more sequelae; delay one or more symptoms; delay one or more sequelae; ameliorate one or more symptoms; ameliorate one or more sequelae; shorten the duration one or more symptoms; shorten the duration of one or more sequelae; reduce the frequency of one or more symptoms; reduce the frequency of one or more sequelae; reduce the severity of one or more symptoms (e.g., reduction of sleep latency); reduce the severity of one or more sequelae; improve the quality of life (e.g., increase of actual sleep time); increase survival; prevent (e.g., suppress, inhibit or delay) a recurrence of the disease or condition; delay a recurrence of the disease or condition; reduce the severity of the disease (e.g., reduction of insomnia disorder severity measured by ISI score); or any combination thereof, e.g., with respect to what is expected in the absence of the treatment with a composition of the present invention.

The term "treatment" also includes prophylaxis or prevention (e.g., suppression, inhibition or delay) of a disease or condition or its symptoms or sequelae thereof. Prophylaxis refers to a therapeutic or course of action used to prevent, inhibit, suppress, reduce the risk, reduce the occurrence or delay the onset of a disease or condition, or to prevent, inhibit, suppress, or delay a symptom associated with a disease or condition.

Sleep disorders are a group of conditions that affect a person's ability to fall asleep or stay asleep, or cause abnormal behaviors during sleep. Non-limiting examples of sleep disorders include insomnia (acute or chronic), sleep apnea, occasional sleeplessness, restless leg syndrome, narcolepsy, parasomnias (e.g., sleepwalking or night terrors), REM sleep behavior disorder, circadian rhythm disorders (e.g., jet lag or shift work disorder), bruxism (teeth grinding), sleep-related movement disorders (e.g., periodic limb movement disorder), or sleep-related breathing disorders. These disorders can significantly impact a person's health, mood, and daytime functioning, and often require medical attention for proper diagnosis and treatment. In an embodiment, the sleep disorder is insomnia disorder.

The term "insomnia disorder", previously called "primary insomnia", relates to a sleep disorder characterized by sleep continuity disturbance, i.e., difficulty initiating and/or maintaining sleep. Insomnia can be classified as acute or chronic. Acute insomnia typically occurs on at least 3 days per week for anywhere between 1 week and 3 months, while chronic insomnia lasts for at least three months and can be caused by a variety of factors, including medical conditions, mental health disorders, medications, and lifestyle factors.

The symptoms of insomnia can include difficulty falling asleep, waking up frequently during the night, waking up too early in the morning, feeling tired upon waking, feeling fatigued during the day, difficulty concentrating, irritability, and mood disturbances.

In some embodiments, the administration of the composition of the invention results in at least one outcome (i.e., effect) selected from the group consisting of:
- a reduction in the insomnia severity (e.g., measured by ISI score);
- a reduction in sleepiness (e.g., measured by ESS score);
- a reduction in sleep latency;
- an increase in total sleep time;
- a reduction of periods of wakefulness (WASO);
- an increase of actual sleep time;
- an increase of bedtime; and
- an improvement of sleep efficiency.

The Insomnia Severity Index (ISI) is a brief self-report questionnaire used to assess the severity of insomnia. It consists of seven questions that ask about the frequency, severity, and impact of insomnia symptoms, as well as satisfaction with current sleep patterns. The ISI is a widely used tool in both clinical and research settings to assess insomnia severity and to monitor treatment outcomes. Each question is scored on a 0-4 scale, with total scores ranging from 0-28, which are interpreted as follows:
0-7: No clinically significant insomnia
8-14: Subthreshold insomnia
15-21: Clinical insomnia of moderate severity
22-28: Clinical insomnia of severe severity

Higher scores indicate more severe insomnia. The ISI is a useful tool for healthcare professionals to assess the severity of insomnia and to monitor treatment progress over time. It can also help individuals better understand their own sleep patterns and provide a starting point for discussions about potential treatment options.

In clinical study of EXAMPLE 1, the ISI was measured at T0 (baseline) and T1 (end of treatment, day 7). After 7 days of treatment, the ISI was improved in *Buenas Noches Total* group, with an ISI decline rate of 53% compared to baseline. Further, the improvement was 60.6% greater for *Buenas Noches Total* group compared to *Buenas Noches* group (from 33% reduction to 53% reduction). A reduction of ISI score correlates with an improvement of insomnia severity.

Accordingly, in an embodiment, the composition of the invention (e.g., *Buenas Noches Total)* causes a reduction in the insomnia severity measured by ISI score. Particularly, the reduction in ISI score after 7 days of treatment with the composition of the invention is of at least about 35%, 40%, 45%, 50%, or 55% compared to baseline, and particularly of about 53%. Alternatively, the reduction in ISI score after 7 days of treatment with the composition of the invention is of at least about 40%, 45%, 50%, 55%, 60%, or 65% compared to a subject treated with a composition without glycine (e.g., *Buenas Noches),* and more particularly of about 60.6%.

The Epworth Sleepiness Scale (ESS) is a self-report questionnaire used to assess the level of daytime sleepiness in individuals. It consists of eight questions that ask about the likelihood of dozing off or falling asleep in different situations, such as sitting and reading, watching TV, or sitting in a car. Each question is scored on a scale of 0-3, with a maximum total score of 24 interpreted as follows:
0-10: Normal range of daytime sleepiness
11-12: Borderline range of daytime sleepiness
13-15: Abnormal range of daytime sleepiness
16-24: Severe range of daytime sleepiness

Higher scores indicate a higher level of daytime sleepiness. The ESS is a quick and easy tool to use in both clinical and research settings to assess daytime sleepiness. The ESS is often used to assess the level of sleepiness in individuals with sleep disorders such as obstructive sleep apnea or narcolepsy. It can also be used to assess the effectiveness of treatments for sleep disorders or to monitor changes in sleepiness levels over time.

In clinical study of EXAMPLE 1 the ESS was measured at T0 (baseline) and T1 (end of treatment, day 7). After 7 days of treatment, the ESS was improved in *Buenas Noches Total* group, with an ESS decline rate of 24% compared to baseline. Further, the improvement was 380% greater for *Buenas Noches Total* group compared to *Buenas Noches* group (from 5% reduction to 24% reduction). A reduction of ESS score correlates with an improvement of sleepiness.

Accordingly, in an embodiment, the composition of the invention (e.g., *Buenas Noches Total)* causes a reduction in the sleepiness measured by ESS score. Particularly, the reduction in ESS score after 7 days of treatment with the composition of the invention is of at least about 10%, 15%, 20%, or 25% compared to baseline, particularly of about 24%. Alternatively, the reduction in EES score after 7 days of treatment with the composition of the invention is of at least about 50%, 100%, 150%, 200%, 250%, 300%, 350% or 400% compared to a subject treated with a composition without glycine (e.g., *Buenas Noches),* and more particularly of about 380%.

Sleep latency is the amount of time it takes for a person to fall asleep after they have gone to bed (bedtime) and tried to fall asleep. It is a measure of the time it takes to transition from a state of wakefulness to sleep. The average sleep latency is typically around 10-20 minutes for healthy adults. In clinical settings, sleep latency is often measured as part of a sleep study or polysomnography, where electrodes are placed on the scalp, face, and other parts of the body to measure brain activity, eye movement, muscle tone, heart rate, and breathing patterns during sleep. Sleep latency can also be measured at home using a sleep diary or actigraphy, which tracks movements during sleep.

In clinical study of EXAMPLE 2, the sleep latency was measured at T0 (mean of data from previous 7 days without treatment) and on each day of treatment (from day 1 to day 7). In this case, the sleep latency was measured by Kronohealth device (Ambulatory Monitoring of Sleep). After 7 days of treatment, *Buenas Noches Total* reduced the sleep latency in 25 minutes (reduction of 74% in comparison with baseline, sleep latency of 9 minutes *versus* 34 minutes in initial time). Moreover, the reduction of sleep latency was of about 6 minutes compared to *Buenas Noches* group (comparison between reduction of 19 minutes with *Buenas Noches versus* reduction of 25 minutes with *Buenas Noches Total).* Therefore, the reduction was 32.14% greater for *Buenas Noches Total* group compared to *Buenas Noches* group (from 56% reduction to 74% reduction).

Accordingly, in an embodiment, the composition of the invention (e.g., *Buenas Noches Total)* causes a reduction in the sleep latency. Particularly, the reduction in sleep latency after 7 days of treatment with the composition of the invention is of at least about 60%, 70% or 80% compared to baseline, particularly of about 74%. Particularly, the sleep latency after 7 days of treatment with the composition of the invention is reduced by 20 minutes, 21 min, 22 min, 23 min, 24 min, 25 min, or 26 min compared to baseline, particularly by 25 minutes. Alternatively, the reduction in sleep latency after 7 days of treatment with the composition of the invention is of at least about 10%, 20%, 30%, or 40% compared to a subject treated with a composition without glycine (e.g., *Buenas Noches),* and more particularly of about 32.14%. Particularly, the sleep latency after 7 days of treatment with the composition of the invention is reduced by 1 minutes, 2 min, 3 min, 4 min, 5 min, 6 min, or 7 min compared to a subject treated with a composition without glycine (e.g., *Buenas Noches),* particularly by 6.2 minutes.

Wake after sleep onset (WASO) is also an important reported parameter, which refers to the periods of wakefulness occurring after the defined sleep onset. This parameter measures wakefulness excluding wakefulness occurring before sleep onset. The WASO time better reflects the fragmentation of sleep and allows to obtain the calculation of the actual sleep time (difference between total sleep time and WASO). The total sleep time (min) is the total time in bed, without sleep latency, awakenings or microawakenings; the WASO (min) is the wake time (awakenings + microawakenings); and the actual sleep time (min) is the difference of (total sleep period) - (WASO).

In clinical study of EXAMPLE 2 the total sleep time, the WASO and the actual sleep time were also measured at T0 (mean of data from previous 7 days without treatment) and on each day of treatment (from day 1 to day 7), all measured by Kronohealth device. After 7 days of treatment, *Buenas Noches Total* increased the actual sleep time by 83 minutes compared to baseline (a 25.6% increase). Particularly, the actual sleep time was 34 minutes greater for *Buenas Noches Total* group compared to *Buenas Noches* group (from 49 min to 83 min), which represents a 71.8% increase (from 14.9% increase to 25.6% increase).

Accordingly, in an embodiment, the composition of the invention (e.g., *Buenas Noches Total)* causes an increase in the actual sleep time. Particularly, the increase in actual sleep time after 7 days of treatment with the composition of the invention is of at least about 16%, 18%, 20% or 25% compared to baseline, particularly of about 25.6%. Particularly, the actual sleep time after 7 days of treatment is increased by 55 minutes, 60 min, 65 min, 70 min, 75 min, or 80 min compared to baseline, particularly by 83 minutes. Alternatively, the increase in actual sleep time after 7 days of treatment with the composition of the invention is of at least about 50%, 60%, 70%, or 80% compared to a subject treated with *Buenas Noches,* and more particularly of about 71.8%. Particularly, the actual sleep time after 7 days of treatment with the composition of the invention is increased by 10 minutes, 15 min, 20 min, 25 min, 30 min, or 35 min compared to a subject treated with *Buenas Noches,* particularly by 34 minutes.

In another embodiment, the composition of the invention (e.g., *Buenas Noches Total)* causes an increase in total sleep time. In another embodiment, the composition of the invention (e.g., *Buenas Noches Total*) causes a reduction in periods of wakefulness (WASO). In another embodiment, the composition of the invention (e.g., *Buenas Noches Total*) causes an increase in bedtime.

Sleep efficiency is another important parameter that refers to the percentage of total time in bed. In clinical study of EXAMPLE 2 the sleep efficiency was also measured at T0 (mean of data from previous 7 days without treatment) and on each day of treatment (from day 1 to day 7), all measured by Kronohealth device. After 7 days of treatment, *Buenas Noches Total* group had a sleep efficiency of 93%, which represents a 14.8% increase compared to baseline (from 81% at baseline to 93% at day 7). Further, the increase was about 3.3% greater for *Buenas Noches Total* group compared to *Buenas Noches* group (from 90% to 93% of sleep efficiency) at day 7.

Accordingly, in an embodiment, the composition of the invention (e.g., *Buenas Noches Total)* results in an improvement in sleep efficiency. Particularly, the improvement in sleep efficiency after 7 days of treatment with the composition of the invention is of at least about 12%, 13%, 14% or 15% compared to baseline, particularly of about 14.8%. Alternatively, the improvement in sleep efficiency after 7 days of treatment with the composition of the invention is of at least about 1%, 2%, 3%, 4%, or 5% compared to a subject treated with *Buenas Noches,* and more particularly of about 3.3%. In another embodiment, the composition of the invention (e.g., *Buenas Noches Total)* has a sleep efficiency of 93% after 7 days of treatment.

Considering all the parameters, the present invention provides compositions for use in the treatment, prevention, or amelioration of sleep disorders, wherein the composition results in at least one outcome selected from the group consisting of:
i) reduces the insomnia severity measured by ISI score;
ii) reduces the sleepiness measured by ESS score;
iii) reduces the sleep latency;
iv) increases the actual sleep time; and
v) improves the sleep efficiency,

compared to baseline (i.e., before treatment), or
compared to a subject treated with the same composition without glycine (e.g., treated with *Buenas Noches).*

In an embodiment, the composition:
i) reduces the insomnia severity by reducing the ISI score in about 50%, particularly 53%;
ii) reduces the sleepiness by reducing the ESS score in about 20%, particularly 24%;
iii) reduces the sleep latency in about 70%, particularly 74% (or alternatively in about 25 minutes, particularly 25 minutes);
iv) increases the actual sleep time in about 25%, particularly 25.6% (or alternatively in about 80 minutes, particularly 83 minutes); and
v) improves the sleep efficiency in about 14%, particularly 14.8% (or alternatively has a sleep efficiency of about 93%, particularly 93%),
compared to baseline.

In another embodiment, the composition:
i) reduces the insomnia severity by reducing the ISI score in about 65%, particularly 60.6%;
ii) reduces the sleepiness by reducing the ESS score in about 380%, particularly 380%;
iii) reduces the sleep latency in about 30%, particularly 32.14%;
iv) increases the actual sleep time in about 70%, particularly 71.8%; and
v) improves the sleep efficiency in about 3%, particularly 3.5%,
compared to a subject treated with the same composition without glycine.

In some embodiments, the administration of the composition of the invention results in the treatment, prevention or amelioration of at least one symptom of insomnia disorder selected from the group consisting of:
- difficulty falling asleep despite feeling tired;
- waking up frequently during the night;
- waking up too early in the morning and being unable to fall back asleep;
- feeling tired or unrefreshed upon waking up in the morning;
- daytime sleepiness or fatigue;
- difficulty concentrating or focusing during the day;
- irritability, mood changes, or anxiety related to sleep;
- increased errors or accidents due to lack of sleep; and
- headaches, gastrointestinal problems, or other physical symptoms related to lack of sleep.

### Products comprising the composition

Another aspect of the invention relates to a nutraceutical product, a food supplement, a medical food, a food product, a pharmaceutical product, or a veterinary product, comprising an effective amount of any of the compositions disclosed herein together with appropriate amounts of acceptable excipients. Alternatively, the composition of the present invention is in the form of a nutraceutical product, a food supplement, a medical food, a food product, a pharmaceutical product, or a veterinary product and the composition comprises appropriate amounts of acceptable excipients.

It is understood that these products include any composition that comprises an active ingredient, e.g., the active ingredients described herein including glycine, together with acceptable excipients. The term "acceptable excipients" as used herein pertains to compounds, materials, compositions, and/or administration forms which are suitable for use in contact with the tissues of a subject (e.g., human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard nutraceutical/food/pharmaceutical texts, i.e., the excipients are pharmaceutically acceptable excipients, nutraceutically acceptable excipients or food/edible acceptable excipients.

The product can adopt different forms or names depending on the product approval route and also depending on the country. In an embodiment, the composition is in the form of pharmaceutical composition, and particularly in the form of medicament. In another embodiment, the composition is in the form of medical food. The terms "medical food" or "food for special medical purposes (FSMP)" are used in some countries to refer to a food specially formulated and intended for the dietary management of a disease that has distinctive nutritional needs that cannot be met by normal diet alone. They are defined in regulations such as the Food and Drug Administration's 1988 Orphan Drug Act Amendments in the United States, and the Commission Directive 1999/21/EC in Europe.

In an embodiment, the composition is in the form of nutraceutical composition, and particularly in the form of food supplement. A food supplement is also known as dietary supplement or nutritional supplement. This is a preparation or product intended to supplement the diet, made from compounds usually used in foodstuffs, which provide nutrients or beneficial ingredients that are not usually ingested in the normal diet or may not be consumed in sufficient quantities. Food supplements are usually sold "over the counter", i.e., without prescription. A food supplement can adopt the form of a pill, a gummy, a capsule, a tablet or a powder, and comprises acceptable excipients. In another embodiment, the composition is in the form of food product, which is fortified with some nutrients (e.g., a bar or yoghurt).

The composition according to the invention (e.g., a food supplement) can be administered as such, can be mixed with a suitable drinkable liquid, such as water, yoghurt, milk or fruit juice, or can be mixed with solid or liquid food. The composition (e.g., the food supplement) can be in the form of tablets or lozenges, pills, capsules, granules, softgels, gels, powders, suspensions, sachets, sweets, gummies, chewing gums, creams, gels, bars, liquids, solutions, syrups, effervescent tablets, sucking tablets, sublingual tablets, sprays, aerosols, emulsions, film preparations, usually in the form of a unit dose.

In particular embodiments, the food supplement comprising the composition of the invention is administered in the form of tablets (compressed form of the supplement, often containing binders and fillers), gels (gelatinous form easy to swallow and can be absorbed quickly by the body), sprays (liquid supplements that are sprayed into the mouth) or gummies (chewable supplements that are often formulated to taste like candy), manufactured in conventional processes of preparing food supplements. Thus, in some embodiments, the product is administered in the form of a tablet, a gel, a spray or a gummy.

As explained herein, the products of the invention comprise an effective amount of any of the compositions disclosed herein together with appropriate amounts of acceptable excipients. The terms "excipient" and "carrier" are used interchangeably and refer to an inert substance added to facilitate administration of a compound.

In some embodiments, excipients are selected, without limitation, from the group consisting of: fillers/diluents/bulking agents, binders, antiadherents, disintegrants, coatings, anti-caking agents, antioxidants, lubricants, sweeteners, flavors, colours, tensides, stabilizers, control-release agents, antimicrobial preservatives, acidifying agents, gelling agents, suspending agents, cosolvents, emulsifying agents and other classes of acceptable excipients.

In an embodiment, the excipient is a filler or a diluent. Fillers or diluents are selected, without limitation, from the group consisting of: inulin, oligofructose, pectin, modified pectin, microcrystalline cellulose, lactose, starch, maltodextrin, saccharose, glucose, fructose, isomalt (hydrogenated isomaltulose), mannitol, xylitol, sorbitol, non-crystallizing sorbitol, calcium carbonate, dicalcium phosphate, tricalcium phosphate, other inert inorganic and organic acceptable fillers, and mixtures of these substances.

In an embodiment, the excipient is a binder. Binders are used in solid dosage forms, e.g., to hold the ingredients in a tablet together, to ensure that tablets and granules can be formed with required mechanical strength, and to give volume to low active dose tablets. Binders in solid dosage forms like tablets are selected, without limitation, from the group consisting of: lactose, sucrose, corn (maize) starch, starches, modified starches, gum arabic (acacia gum, arabic gum, gum acacia) microcrystalline cellulose, modified cellulose (e.g., hydroxypropyl methylcellulose (HPMC), hydroxypropyl cellulose, carboxymethyl cellulose and hydroxyethyl cellulose), other water soluble cellulose ethers, polyvinylpyrrolidone (PVP) also known as povidone, polyvinyl alcohol, polyethylene glycol, sorbitol, maltitol, xylitol and dibasic calcium phosphate, other suitable acceptable binders, or mixtures of these substances.

In an embodiment, the excipient is an antiadherent. Antiadherents are used to reduce the adhesion between the powder (granules) and the punch faces and thus prevent sticking to tablet punches. They are also used to help protect tablets from sticking. Antiadherents are particularly magnesium stearate or polyethylene glycol.

In an embodiment, the excipient is a disintegrant or a superdisintegrant. As disintegrants and superdisintegrants in solid dosage forms like tablets and capsules, the following substances, without limitation, particularly used: cross-linked polyvinylpyrrolidone, starches, sodium croscarmellose, sodium starch glycolate, sodium carboxymethyl cellulose, calcium carboxymethyl cellulose, sodium glycolate (e.g., EXPLOTAB^{®}), formaldehyde-casein, other suitable acceptable disintegrant and superdisintegrant, or their mixtures.

In an embodiment, the excipient is a coating. Coatings in the case of solid dosage forms, such as tablets and granules for capsules filling, protect the ingredients from deterioration by moisture in the air, make large, unpleasant-tasting tablets easier to swallow and/or in the case of enteric coatings, ensure intact passage through a strong acidic medium of gastric juice (pH around 1), and release in duodenum or ileum (small intestine). In an embodiment, for most coated tablets, a cellulose ether hydroxypropyl methylcellulose (HPMC) film coating is used. In other embodiments, other coating materials are used, e.g., synthetic polymers and co-polymers like polyvinylacetate phthalate (PVAP); co-polymers of methyl acrylate-metacrylic acid; co-polymers of methyl metacrylate-metacrylic acid; shellac, corn protein zein or other polysaccharides (e.g., microcrystalline cellulose); waxes or wax-like substances such as beeswax, stearic acid, or carnauba wax; higher fatty alcohols like cetyl or stearyl alcohol; solid paraffin; glycerol monostearate; glyceryl distearate, or their combinations. Particularly, capsules are coated with gelatin or hydroxypropyl methylcellulose.

Enteric coatings control the rate of active ingredient release and determine where the ingredient is released in the digestive tract. In some embodiments, materials used for enteric coatings include fatty acids, waxes, shellac, plastics, and plant fibers and their mixtures, also in combination with other above mentioned coatings.

In an embodiment, the excipient is an anticaking agent. An anticaking agent is an additive placed in powdered or granulated materials to prevent the formation of lumps (caking) and for easing packaging, transport, and consumption. As anti-caking agents in solid dosage forms like tablets, capsules, or powders, the following are particularly used: magnesium stearate, colloidal silicon dioxide, talc, other acceptable anticaking agents, or their mixtures.

In an embodiment, the excipient is an antioxidant. Antioxidants are often used in compositions to protect the active ingredients from oxidative degradation. Particular antioxidants used in compositions include, without limitation, ascorbic acid (vitamin C), alpha-tocopherol (vitamin E), butylated hydroxyanisole (BHA), butylated hydroxytoluene (BHT), propyl gallate, and citric acid.

In an embodiment, the excipient is a lubricant. Lubricants are used in solid dosage forms, in particular in tablets and capsules, to prevent ingredients from clumping together and from sticking to the tablet punches or capsule filling machine, and also in hard capsules. As lubricants talc or silica, polyethylene glycol, calcium silicate and fats, e.g., vegetable stearin, magnesium stearate or stearic acid, and mixtures thereof, are particularly used lubricants in tablets or hard gelatin capsules.

In an embodiment, the excipient is a sweetener. Sweeteners are added to make the ingredients more palatable, especially in solid dosage forms, e.g., chewable tablets, as well as in liquids dosage forms, like cough syrup. In particular, sweeteners are selected from artificial, natural or synthetic or semisynthetic sweeteners. Non-limiting examples of sweeteners are aspartame, acesulfame potassium, cyclamate, sucralose, saccharine, maltodextrin, polyols (e.g., isomalt (hydrogenated isomaltulose), sorbitol, mannitol), sugars (e.g., fructose), steviol glycosides, or any mixture thereof.

In an embodiment, the excipient is a flavor. Flavors can be used to mask unpleasant tasting active ingredients in any dosage form. Flavorings can be natural (e.g., fruit extract) or artificial. For example, to improve: (1) a bitter product, mint, cherry or anise can be used; (2) a salty product, peach or apricot or liquorice can be used; (3) a sour product, raspberry; and (4) an excessively sweet product, vanilla.

In an embodiment, the excipient is a stabilizer. A stabilizer is a type of excipient that is added to a composition to help maintain the chemical and physical stability of the active ingredients during storage and use. An example of stabilizer is hydroxypropyl cellulose.

In an embodiment, the excipient is a controlled-release agent. The function of a controlled-release agent is to regulate the release of active ingredients over an extended period. It helps to maintain a steady concentration of the active ingredient in e.g., the bloodstream, which can improve the effectiveness of the treatment and reduce side effects. Controlled-release agents can take many forms, such as polymers, coatings, or matrix systems. These agents can be designed to release the active ingredient in a variety of ways, such as a sustained release or a delayed release. Non-limiting examples of control-release agents are hydroxypropyl methylcellulose and carboxymethylcellulose.

In an embodiment, the excipient is a preservative. An antimicrobial preservative is a type of excipient that prevents the growth of microorganisms, such as bacteria, fungi, and viruses, in the product, and helps to extend the shelf life of the product, improve its stability, and ensure that it is safe to use. Particular antimicrobial preservatives used include benzalkonium chloride, chlorhexidine, phenol, sodium benzoate, potassium sorbate, sorbic acid and glycerin.

In an embodiment, the excipient is an acidifying agent. Acidifying agents can be used to lower the pH of the formulation for e.g., preservation, solubility, or taste reasons. Particular acidifying agents include citric acid, tartaric acid, and acetic acid.

In an embodiment, the excipient is a gelling agent. A gelling agent is a substance that can convert a liquid into a gel or semi-solid state. Gelling agents are used to thicken the formulation and provide a consistent texture. They can also improve the stability, bioavailability, and controlled release of active ingredients. Gelling agents are commonly used in topical formulations such as gels, creams, and ointments, as well as oral formulations such as gummies, jellies, syrups, suspensions, and soft gel capsules. Some examples of gelling agents include agar, agar-agar, carrageenan, xanthan gum, guar gum, gum arabic (acacia gum, arabic gum, gum acacia), gelatin, pectin and starches.

In an embodiment, the excipient is a suspending agent. A suspending agent is a type of excipient that is used to suspend insoluble particles in a liquid dosage form, such as a suspension or emulsion. It helps to prevent settling of the particles and ensures a uniform distribution of the active ingredient throughout the liquid. Suspending agents are particularly important for compositions that are poorly soluble or that are administered orally in a liquid form. Particular suspending agents include natural and synthetic polymers, such as cellulose derivatives, acrylic polymers, and polysaccharides (e.g., xanthan gum, guar gum, gum arabic (acacia gum, arabic gum, gum acacia)), starches, as well as clays, such as bentonite and magnesium aluminum silicate.

In an embodiment, the excipient is a cosolvent. A cosolvent is a solvent that is added to a composition to enhance the solubility of a particular ingredient. Cosolvents can help to dissolve poorly soluble or insoluble compounds, and thus improve the bioavailability and efficacy of the active ingredient. Particular cosolvents used include ethanol, propylene glycol, polyethylene glycol, glycerol (glycerin), and surfactants such as polysorbate (Tween^{®}) and polyethoxylated castor oil (Kolliphor^{®}).

In an embodiment, the excipient is an emulsifier. An emulsifying agent or emulsifier is a type of excipient to help mix two immiscible liquids, such as oil and water, and create a stable emulsion. They help to improve the bioavailability and absorption of lipophilic or hydrophilic active ingredients. They work by reducing the surface tension between the two liquids, which allows them to form a stable mixture. Some examples of emulsifying agents include trisodium citrate, lecithin, polysorbate 80, and sodium lauryl sulfate.

Selection of the excipients and the most appropriate methods for formulation in view of the particular product form of the composition is within the scope of ordinary persons skilled in the art of nutraceutical/food/pharmaceutical technology.

For example, if the composition according to the invention is administered as a tablet, the selected excipients would include, e.g., binders, fillers, disintegrants, lubricants, fillers/diluents, anti-caking agents, antiadherents, coating agents, stabilizers, control-release agents, colouring agents, flavouring agents or sweeteners. Accordingly, in an embodiment, the tablet comprises at least one of the following excipients: silicon dioxide, magnesium stearate, microcrystalline cellulose, hydroxypropyl cellulose, hydroxypropyl methylcellulose, carboxymethylcellulose, starches, sodium croscarmellose, polyethylene glycol, polyvinylpyrrolidone, maltodextrin, polyols (e.g., isomalt (hydrogenated isomaltulose), sorbitol, or mannitol), polyvinyl alcohol, calcium carbonate, calcium silicate, talc, tricalcium phosphate, dicalcium phosphate, and gum arabic (acacia gum, arabic gum, gum acacia).

If the composition according to the invention is administered as a spray, the selected excipients can include, e.g., gelling agents, antimicrobial preservatives, acidifying agents, antioxidants, suspending agents, sweeteners, fillers or cosolvents. Accordingly, in an embodiment, the spray comprises at least one of the following excipients: sodium benzoate, potassium sorbate, citric acid, xanthan gum, guar gum, gum arabic, sucralose, polyols (e.g., isomalt (hydrogenated isomaltulose), sorbitol, or mannitol), steviol glycosides, fructose, starches, pectin, and glycerin.

If the composition according to the invention is administered as a gummy, the selected excipients can include, e.g., gelling agents, suspending agents, fillers, binders, antimicrobial preservatives, cosolvents, coating agents, disintegrants, emulsifying agents, acidifying agents, antioxidants, or sweeteners. Accordingly, in an embodiment, the gummy comprises at least one of the following excipients: pectin, gelatin, xanthan gum, guar gum, gum arabic, agar-agar, polyols (e.g., isomalt (hydrogenated isomaltulose), sorbitol, or mannitol), glycerin, carnauba wax, starches, trisodium citrate, citric acid, sucralose, steviol glycosides, and fructose.

The product of the invention can be in the format of a bilayer or tri-layer, e.g., a bilayer or tri-layer tablet or gummy. A bilayer or tri-layer product is a type of product, e.g., tablet/gummy, that has two or three layers of different formulations or active ingredients. Each layer is separated by a barrier, which can be a physical barrier or a coating that controls the release of the ingredients. Bilayer or tri-layer tablets are often used in food supplements to provide a combination of complementary ingredients that need to be released at different times or in different parts of the digestive system.

In an embodiment, the bilayer ortri-layertablet or gummy comprises an external layer and an internal layer. Alternatively, the bilayer or tri-layer tablet or gummy comprises a lower layer and an upper layer. Said bilayer or tri-layer tablets or gummies can further comprise a coating. Particularly, the external layer comprises melatonin.

In an embodiment, the lower layer comprises at least one of the following components: valerian root dry extract (e.g., *Valeriana officinalis* L.), GABA, *Passiflora* dry extract (e.g., *Passiflora incarnata* L.), *Californian poppy* dry extract (e.g., *Eschscholzia californica* Cham.), silicon dioxide, calcium silicate, magnesium stearate, microcrystalline cellulose, hydroxypropyl cellulose, vitamin B6 (e.g., pyridoxine hydrochloride), vitamin B3 (e.g., nicotinamide), and glycine.

Particularly, the lower layer comprises at least one of the following components in the following amounts: valerian root dry extract (e.g., *Valeriana officinalis* L.) in a range of between 20 and 200 mg, GABA in a range of between 50 and 250 mg, *Passiflora* dry extract (e.g., *Passiflora incarnata* L.) in a range of between 20 and 200 mg, *Californian poppy* dry extract (e.g., *Eschscholzia californica* Cham.) in a range of between 20 and 200 mg, silicon dioxide in a range of between 1 and 15 mg, calcium silicate in a range of between 1 and 15 mg, magnesium stearate in a range of between 1 and 10 mg, microcrystalline cellulose in a range of between 20 and 150 mg, hydroxypropyl cellulose in a range of between 10 and 100 mg, pyridoxine hydrochloride (vitamin B6) in a range of between 0.5 and 5 mg, nicotinamide (vitamin B3) in a range of between 3 and 30 mg, and glycine in a range of between 25 and 150 mg.

More particularly, the lower layer comprises at least one of the following components in the following amounts: about 100 mg of valerian root dry extract (e.g., *Valeriana officinalis* L.), about 100 mg of GABA, about 75 mg of *Passiflora* dry extract (e.g., *Passiflora incarnata* L.), about 25 mg of *Californian poppy* dry extract (e.g., *Eschscholzia californica* Cham.), about 6 mg of silicon dioxide, about 3 mg of magnesium stearate, about 82.5 mg of microcrystalline cellulose, about 56 mg of hydroxypropyl cellulose, about 1.4 mg of pyridoxine hydrochloride (vitamin B6), about 16 mg of nicotinamide (Vitamin B3), and about 50 mg of glycine. In another embodiment, the lower layer comprises at least one of the following components in the following amounts: about 100 mg of valerian root dry extract (e.g., *Valeriana officinalis* L.), about 100 mg of GABA, about 75 mg of *Passiflora* dry extract (e.g., *Passiflora incarnata* L.), about 25 mg of *Californian poppy* dry extract (e.g., *Eschscholzia californica* Cham.), about 74.5 mg of microcrystalline cellulose, about 56 mg of hydroxypropyl cellulose, about 1.7 mg of pyridoxine hydrochloride (vitamin B6), about 16 mg of nicotinamide (vitamin B3), about 10.7 mg of magnesium stearate, about 6 mg of calcium silicate, and about 50 mg of glycine.

In another embodiment, the upper layer comprises at least one of the following components: melatonin, silicon dioxide, calcium silicate, magnesium stearate, microcrystalline cellulose, sodium glycolate (EXPLOTAB^{®}), and potato starch.

Particularly, the upper layer comprises at least one of the following components in the following amounts: melatonin in an amount of less than 2 mg, silicon dioxide in a range of between 0.5 and 5 mg, calcium silicate in a range of between 0.5 and 5 mg, magnesium stearate in a range of between 0.1 and 2 mg, microcrystalline cellulose in a range of between 30 and 200 mg, sodium glycolate (EXPLOTAB^{®}) in a range of between 3 and 11 mg, and potato starch in a range of between 3 and 11 mg.

More particularly, the upper layer comprises at least one of the following components in the following amounts: about 1.85 mg of melatonin, about 2 mg of silicon dioxide, about 0.68 mg of magnesium stearate, about 123.5 mg of microcrystalline cellulose, and about 7 mg of sodium glycolate (EXPLOTAB^{®}). In another embodiment, the upper layer comprises at least one of the following components in the following amounts: about 1.85 mg of melatonin, about 2 mg of calcium silicate, about 0.68 mg of magnesium stearate, about 123.5 mg of microcrystalline cellulose, and about 7 mg of potato starch.

In another embodiment, the coating comprises at least one of the following components: hydroxypropyl methylcellulose, microcrystalline cellulose, and stearic acid.

Particularly, the coating comprises at least one of the following components in the following amounts: hydroxypropyl methylcellulose in a range of between 2 and 15 mg, microcrystalline cellulose in a range of between 0.1 and 5 mg, and stearic acid in a range of between 0.1 ang 5 mg.

More particularly, the coating comprises at least one of the following components in the following amounts: about 8 mg of hydroxypropyl methylcellulose, about 1 mg of microcrystalline cellulose, and about 1 mg of stearic acid.

In a particular embodiment, the product of the present invention is in the form of bilayer or tri-layer tablets or gummies comprising a lower layer, an upper layer, and a coating, wherein:
the lower layer comprises at least one of the following components: valerian root dry extract (e.g., *Valeriana officinalis* L.), GABA, *Passiflora* dry extract (e.g., *Passiflora incarnata* L.), *Californian poppy* dry extract (e.g., *Eschscholzia californica* Cham.), silicon dioxide, calcium silicate, magnesium stearate, microcrystalline cellulose, hydroxypropyl cellulose, pyridoxine hydrochloride (vitamin B6), nicotinamide (vitamin B3), and glycine;
the upper layer comprises at least one of the following components: melatonin, silicon dioxide, calcium silicate, magnesium stearate, microcrystalline cellulose, sodium glycolate (EXPLOTAB^{®}), and potato starch; and
the coating comprises at least one of the following components: hydroxypropyl methylcellulose, microcrystalline cellulose, and stearic acid.

The above products include melatonin in the outer layer (upper layer) to help control the timing and rate of release of the hormone into the body, improving its effectiveness in promoting sleep.

In a particular embodiment, the product of the present invention is in the form of bilayer or tri-layer tablets or gummies comprising a lower layer, an upper layer, and a coating, wherein:
the lower layer comprises at least one of the following components in the following amounts: about 100 mg of valerian root dry extract (e.g., *Valeriana officinalis* L.), about 100 mg of GABA, about 75 mg of *Passiflora* dry extract (e.g., *Passiflora incarnata* L.), about 25 mg of *Californian poppy* dry extract (e.g., *Eschscholzia californica* Cham.), about 6 mg of silicon dioxide, about 3 mg of magnesium stearate, about 82.5 mg of microcrystalline cellulose, about 56 mg of hydroxypropyl cellulose, about 1.4 mg of pyridoxine hydrochloride (vitamin B6), about 16 mg of nicotinamide (vitamin B3), and about 50 mg of glycine;
the upper layer comprises at least one of the following components in the following amounts: about 1.85 mg of melatonin, about 2 mg of silicon dioxide, about 0.68 mg of magnesium stearate, about 123.5 mg of microcrystalline cellulose, and about 7 mg of sodium glycolate (EXPLOTAB^{®}); and the coating comprises at least one of the following components in the following amounts: about 8 mg of hydroxypropyl methylcellulose, about 1 mg of microcrystalline cellulose, and about 1 mg of stearic acid.

In another particular embodiment, the product of the present invention is in the form of bilayer or tri-layer tablets or gummies comprising a lower layer, an upper layer, and a coating, wherein:
the lower layer comprises at least one of the following components in the following amounts: about 100 mg of valerian root dry extract (e.g., *Valeriana officinalis* L.), about 100 mg of GABA, about 75 mg of *Passiflora* dry extract (e.g., *Passiflora incarnata* L.), about 25 mg of *Californian poppy* dry extract (e.g., *Eschscholzia californica* Cham.), about 74.5 mg of microcrystalline cellulose, about 56 mg of hydroxypropyl cellulose, about 1.7 mg of pyridoxine hydrochloride (vitamin B6), about 16 mg of nicotinamide (vitamin B3), about 10.7 mg of magnesium stearate, about 6 mg of calcium silicate, and about 50 mg of glycine;
the upper layer comprises at least one of the following components in the following amounts: about 1.85 mg of melatonin, about 2 mg of calcium silicate, about 0.68 mg of magnesium stearate, about 123.5 mg of microcrystalline cellulose, and about 7 mg of potato starch; and
the coating comprises at least one of the following components in the following amounts: about 8 mg of hydroxypropyl methylcellulose, about 1 mg of microcrystalline cellulose, and about 1 mg of stearic acid.

In an embodiment, the product has a final weight (i.e., total weight) of between 100 mg and 1 g, between 200 mg and 900 mg, between 300 mg and 800 mg, between 400 and 700 mg, or particularly of between 500 and 700 mg. In another embodiment, the product has a final weight of about: 500 mg, 550 mg, 600 mg, or 650 mg, 700 mg; more particularly of about: 600 mg, 610 mg, 620 mg, 630 mg, 640 mg, 650 mg, 660 mg, 670 mg, 680 mg, 690 mg or 700 mg. Particularly, the product has a final weight of 660 mg.

### Administration and dosage

In an embodiment, the composition is administered orally.

Daily dosages and therapeutic regimens will be determined by the skilled in the art. For example, daily dosage may be higher during the treatment of active symptoms or may be lower for maintenance or prophylactic purposes.

Particularly for the compositions of the invention, the frequency of administration is a daily e.g., tablet or gummy, and the duration of treatment can be from one day to the number of days necessary to observe an improvement in the quality of sleep.

Accordingly, in an embodiment, the composition is administered once daily, particularly before bedtime. Particularly, the composition is administered 120 min, 90 min, 75 min, 60 min, 45 min, 30 min, 20 min, 10 min, or 5 min before bedtime, and more particularly 30 min before bedtime. Thus, the glycine of the composition is administered at a dosage of about 25 mg/day, about 30 mg/day, about 40 mg/day, about 50 mg/day, about 60 mg/day, about 70 mg/day, about 80 mg/day, about 90 mg/day, about 100 mg/day, about 110 mg/day, about 120 mg/day, about 130 mg/day, about 140 mg day, or about 150 mg/day, and particularly about 50 mg/day.

In another embodiment, the composition is administered once daily for at least 1 day, for at least 2 days, for at least 3 days, for at least 4 days, for at least 5 days, for at least 6 days, for at least 7 days, for at least 15 days, for at least 30 days, for at least 2 months, for at least 6 months, or for at least 1 year.

Additionally, the administration can be chronic or intermittent, as deemed appropriate by the supervising practitioner, particularly in view of any change in the disease state or any undesirable side effects. "Chronic" administration refers to administration of the composition in a continuous manner while "intermittent" administration refers to treatment that is done with interruption.

### Methods of preparation

Regarding the preparation of the formulations of the present invention is within the scope of ordinary person skilled in the art and will depend upon the final dosage formulation.

For instance, and without limitation, when the final dosage form is an oral solid one, such as tablets, capsules, powder, granules, or oral suspensions, the process for preparation of solid dosage forms includes homogenization of: (1) the active ingredient(s); (2) with one or more excipients to form homogeneous mixture which is, according to requirements, subjected to e.g. lubrication with e.g., magnesium stearate or other lubricants yielding final dosage form of powder. Such homogeneous powder is filled into ordinary gelatin capsules or, alternatively, into gastro-resistant capsules. In the case of tablets, they are manufactured by direct compression or granulation. In the first case, a homogeneous mixture of active ingredients and suitable excipients such as anhydrous lactose, non-crystallizing sorbitol, and others is prepared. In the second case, tablets are processed on the mixture in granulated form. Granules are prepared by a granulation process of the active ingredients of the formulation with suitable fillers, binders, disintegrants, and a small amount of purified water. Such prepared granules are sieved and dried until the water content of <1 % w/w.

Particularly, in the case of a tablet, the granulated mix is manufactured by wet granulation, by spraying the binding liquid directly on the pre-mix in fluid bed. The technique of top spraying can be adopted. The working temperature is adjusted, and the process is finished consuming all the binding liquid. The obtained granulate is sampled to assess the apparent and compacted density, its particle size distribution and porosity. Tablets can be made of several specific formats like monolayer or multilayer tablets, by setting up the machine with different tablet press formats. The powder/granulated mixture is compressed according to pre-established quality specifications. The obtained core is coated and packaged into its primary and secondary packaging material.

Particularly, regarding the process for preparation of a jelly (stick), a gummy (blisters) or a gel, the suspension of excipients is made by single rotor mix procedure in an industrial scale liquid dosage forms tank, by adding the excipients into the main solvent (water), except for the gelling agents that are weighed and set aside until use. After the incorporation, the composition of the present invention and additional solid components, solvents, and co-solvents are mixed up by a mechanical helicoidal stirrer which applies a continuous vigorous stirring. After obtaining the final liquid, the product is sterilized by pasteurization or UHT process and transferred to the packaging lung tank. The obtained yield is calculated before adding the gelling agent. The addition of the gelling agent is executed at a temperature of e.g., minimum 80°C, under continuous and vigorous stirring. The warm liquid is packaged into its primary packaging before the product might start cooling. The process is similar regardless of whether the dosage form is a jelly, a gummy, or a gel.

Regarding the process for preparation of liquid dosage forms (e.g., oral suspension, sprays), it involves homogenization of the active ingredient(s) of the formulation in an inert liquid diluent (filler) such as various vegetable oils like sunflower, soybean or olive oil; oleic acid; oleyl alcohol; liquid polyethylene glycols like PEG 200, PEG 400 or PEG 600; or other inert acceptable liquids. The process further involves treatment of homogeneous mixture with one or more processes selected from the group comprising: (1) stabilization of the formulation, by addition and homogenization of suspension stabilizers like beeswax, colloidal silicon dioxide, etc.; (2) sweetening of the formulation; by addition and homogenization of sweetener; (3) flavoring of the formulation, by addition and homogenization of flavoring. Such forms of the formulation can contain also other excipients or ingredients, usually employed in the art.

### EXAMPLES

### EXAMPLE 1: Efficacy and safety of Buenas Noches and Buenas Noches Total products on night-time sleep and daytime symptoms in patients with primary insomnia. Subjective Sleep Quality according to Insomnia Severity Index (ISI) and Epworth Sleepiness Scale (ESS)

### 1.1 Materials and methods

### 1.1.1 Subjects, study design and conduct

A total of 60 participants (over 18 years of age and of both sexes) with primary insomnia were enrolled. Insomnia disorder was diagnosed according to the 5th edition of the Diagnostic and Statistical Manual of Mental Disorders criteria (insomnia DSM-5 criteria). Eligible participants were randomly assigned to the following groups: placebo (n=20), *Buenas Noches* (n=20) and *Buenas Noches Total* (n=20). A balanced randomization (1:1:1) was carried out, in which the random distribution of each group (treatments or placebo) was ensured. The purpose of randomization was to ensure that each patient had an equal chance of being assigned to either of the two treatment groups. The participants and investigators were blinded to the group allocation.

The present randomized, double-blind, and placebo-controlled study was conducted at Health Area of Don Benito-Villanueva (Badajoz, Spain, 2022). The study was performed in accordance with the Declaration of Helsinki (64th WMA General Assembly, Fortaleza, Brazil, October 2013) and current legislation (Law17/2007 in Spain, about Biomedical Research and Good Clinical Practices guidelines (CPMP/ICH/135/1995). Participants signed informed consent forms after investigators sufficiently explained this clinical trial and received a copy of the consent form.

### 1.1.2 Interventions

Patients were administered one tablet/day of either placebo, *Buenas Noches* or *Buenas Noches Total.* The active ingredients of *Buenas Noches* and *Buenas Noches Total* tablets are listed in Table 1.

**Table 1. Active ingredients of Buenas Noches and Buenas Noches Total tablets.**

| **Composition** | ***Buenas Noches** (mg)** | ***Buenas Noches Total* (mg)** |
|---|---|---|
| Valerian root dry extract (*Valeriana officinalis* L., *Valerianae radix*) | 100 | 100 |
| Passiflora dry extract (*Passiflora incarnata* = *Passiflora edulis, Passiflora herba*) | 100 | 75 |
| Californian poppy dry extract (*Eschscholzia californica*, *Eschscholziae herba*) | 50 | 25 |
| Vitamin B6 | 1.4 | 1.4 |
| Vitamin B3 | 16 | 16 |
| Melatonin | 1.85 | 1.85 |
| GABA | 100 | 100 |
| Glycine | - | 50 |

| | | |
|---|---|---|
| * Batch code: S1 1-0006 | | |

Each tablet contained excipients together with active ingredients, except for placebo tables which were made of only microcrystalline cellulose in the same weight and appearance as the *Buenas Noches* and *Buenas Noches Total* tablets. In the case of *Buenas Noches* and *Buenas Noches Total* tablets, the excipients used were:
- lower layer: 6 mg of silicon dioxide, 3 mg of magnesium stearate, 82.5 mg of microcrystalline cellulose, 56 mg of hydroxypropyl cellulose;
- upper layer: 2 mg of silicon dioxide, 0.68 mg of magnesium stearate, 123.5 mg of microcrystalline cellulose, and 7 mg of sodium glycolate (EXPLOTAB^{®}); and
- coating: 8 mg of hydroxypropyl methylcellulose, 1 mg of microcrystalline cellulose, and 1 mg of stearic acid.

The treatment was continued for 1 week. Posology for each group: 1 tablet at night, half an hour before going to bed.

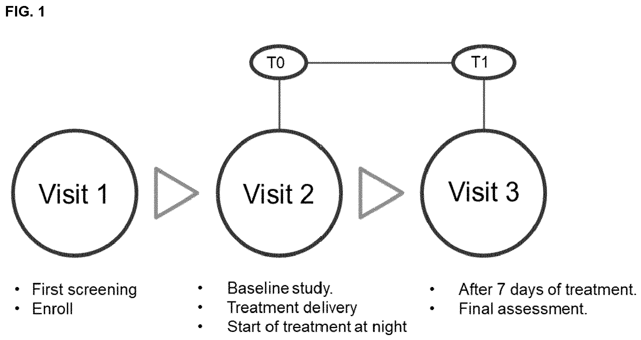

The duration of the study was 7 days per patient with a total of 3 visits: a screening visit (visit one), a visit for the baseline study where either *Buenas Noches*, *Buenas Noches Total* or a placebo was provided to the patients (visit two, T0), and a visit at the end treatment at day 7 (visit three, T1), when the bottles of the administered products (treatments and placebo) were returned to the principal investigator to assess compliance with the posology described in the protocol (see schedule in FIG. 1).

### 1.1.3 Eligibility Criteria

Inclusion criteria: (1) male and female participants aged >18 years. (2) Diagnosis of insomnia disorder according to DSM-5 criteria: difficulties initiating sleep, maintaining sleep, early awakenings, and the inability to fall asleep again; (3) Insomnia duration ≥ 3 months; (4) Voluntary participation and signing of an informed consent form after adequate explanation of the purpose and procedure of this clinical trial.

Exclusion criteria: (1) Intolerance or hypersensitivity to the food supplement, or to any of its components; (2) Treatment with benzodiazepines or non-benzodiazepine hypnotics within the previous 2 weeks or any treatment with psychotropic drugs; (3) Within the past 3 months, sleep disturbance associated with a psychiatric disorder (e.g., major depression, anxiety, dementia, other cognitive impairment, Parkinson's), sleep disturbance secondary to another medical condition (e.g., sleep apnea, circadian rhythm sleep-wake disorder), concomitant use of medications such as psychotropic treatments, neuroleptics, antiepileptics, barbiturates, antidepressants, anxiolytics, lithium, first-generation antihistamines, muscle relaxants, opioid-type analgesics, or treatments used as hypnotics (e.g.,, all benzodiazepines, zopiclone, zolpidem and zaleplon, barbiturates, buspirone and hydroxyzine, tryptophan, melatonin); (4) Treatment with contraceptive hormones, quinolone antibiotics, rifampicin; (5) Any chronic medical condition that is likely to be the cause of the sleep problem (e.g.,, chronic pain, benign prostatic hypertrophy, recent loss of a loved one, stressful events); (6) Medical condition that requires chronic treatment with medications or other substances; (7) Consumption of alcohol as a sleep inducer; (8) Smoking should be avoided during the study as it can reduce the concentration of melatonin; (9) Pregnancy or breastfeeding; (10) Any condition that, in the opinion of the investigators, disables the subject from participating in the study.

### 1.1.4 Primary and secondary endpoints

The primary endpoint of the study was to assess the clinical efficacy of *Buenas Noches* and *Buenas Noches Total* through the evaluation of insomnia severity and sleepiness.

The clinical findings were documented during visits in accordance with the measure and the mean difference in the total Insomnia Severity Index (ISI) and Epworth Sleepiness Scale (ESS) scores pre- and post-intervention between the three groups. ISI and ESS scores are two validated questionnaires:
- ISI score (Insomnia Severity Index): a 7-item questionnaire asking respondents to rate the nature and symptoms of their sleep problems using a Likert-type scale. Responses can range from 0 to 4, where higher scores indicate more acute symptoms of insomnia.

Questions relate to subjective qualities of the respondent's sleep, including the severity of symptoms, the respondent's satisfaction with his or her sleep patterns, the degree to which insomnia interferes with daily functioning, how noticeable the respondent feels his or her insomnia is to others, and the overall level of distress created by the sleep problem.
- ESS score (Epworth Sleepiness Scale): this questionnaire was developed to determine the level of daytime sleepiness in individuals, during different routine situations (excessive daytime sleepiness) (i.e., reading, watching TV, sitting in public, being a car passenger, resting in the afternoon, talking to someone, sitting quietly after lunch, stopping in traffic). It has become one of the most frequently used methods for determining a person's average level of daytime sleepiness. Daytime sleepiness is an important manifestation of sleep disorders, and it impacts the patient's social life and threatens public health and safety. The ESS appears to be a convenient, standardized, and cost-effective way to measure sleepiness in patients who suffer from sleep disorders.

It has 8-item questions, also using a 4-point Likert-type scale. Each item is rated from 0 to 3, with 0 meaning you would never doze or fall asleep in a given situation, and 3 meaning there is a very high chance that you would doze or fall asleep in that situation. These scores are summed to determine a total ESS score, which ranges between 0 and 24. A cut-off value of 10 (ESS total score > 10) is usually considered to detect excessive daytime sleepiness (EDS).

The secondary endpoints were the evaluation of the satisfaction of the treatment, through questionnaires to the participants.

The clinical safety was evaluated through the registration of adverse effects in patients and the identification of those that were device-related adverse effects.

### 1.1.5 Statistical Analysis

Initially, a descriptive and graphic analysis of the main variables under study was carried out. The normality test of the quantitative variables was included for apply parametric or non-parametric tests. If the number of data is greater than 50, we must consider the Kolmogorov-Smirnov statistic for the normality test and if it is less than 50, the Shapiro-Wilk statistic. When the p-value of the normality test is significant (p<0.05) we can accept the hypothesis that the variable does not have a normal distribution. On the other hand, if the p-value is not significant (p>0.05) we would accept the hypothesis that the variable does have a normal distribution.

The independent t-test (Student's T, ANOVA) or Kruskal Wallis-test were used for analysis, depending on the normality of data distribution. Statistical software IBM SPSS version 26.0 was be used to carry out all analyses.

### 1.2 Results

There were no differences in sex ratio of the volunteers assigned to each study group (Placebo group: 50% men, 50% women; *Buenas Noches* group: 50% men, 50% women; *Buenas Noches Total* group: 50% men, 50% women; test of Pearson's chi-square: P = 1.000).

There were also no differences at the start of the study (T0) between volunteers included in the Placebo group, the *Buenas Noches* group and the *Buenas Noches Total* group in age (Kruskal-Wallis H Test: P = 0.995), height (Test Kruskal-Wallis H: P = 0.584), weight (Kruskal-Wallis H Test: P = 0.993), and BMI (Kruskal-Wallis H Test: P = 0.821) (Table 2).

**Table 2. Baseline characteristics of subjects enrolled for the study (mean ± SD).**

| | **Age** | **Height** | **Weight** | **Body mass index (BMI)** |
|---|---|---|---|---|
| **Placebo** (n=20) | 36.30 ± 6.70 | 1.70 ± 0.07 | 69.35± 10.36 | 23.81 ± 2.21 |
| **Buenas Noches** (n=20) | 36.50 ± 7.56 | 1.68 ± 0.05 | 69.60 ± 9.98 | 24.49 ± 2.64 |
| **Buenas Noches Total** (n=20) | 36.30 ± 10.22 | 1.68 ± 0.06 | 68.65 ± 6.17 | 24.35 ± 1.62 |

All participants completed the entire study protocol.

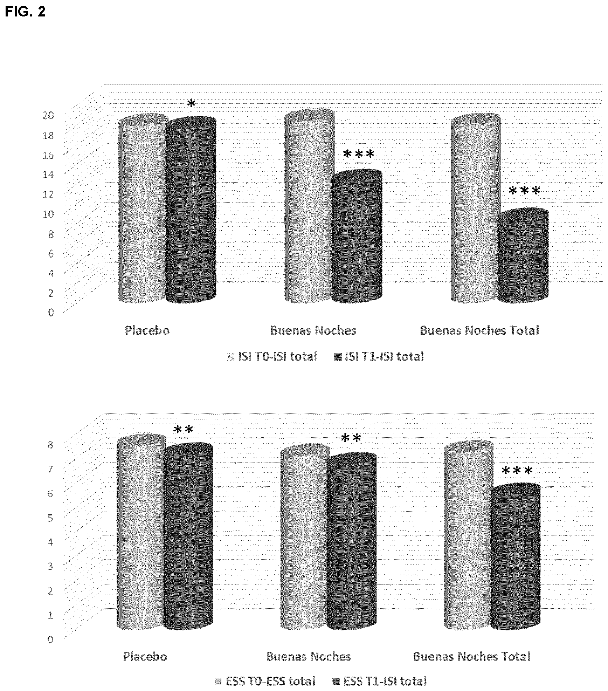

According to the primary endpoint of the study, calculated scores for the ISI and ESS are presented in Table 3 and FIG. 2.

**Table 3. Results obtained for the ISI and ESS scores (mean ± SD) and reduction or ISI and ESS decline rates.**

| | **ISI Total** | | | **ESS Total** | | |
|---|---|---|---|---|---|---|
| | **T0** | **T1** | **Reduction*** | **T0** | **T1** | **Reduction**** |
| **Placebo** (n=20) | 17.95 ± 1.70 | 17.70 ± 1.84 | 1 % | 7.55 ± 0.83 | 7.20 ± 0.70 | 5% |
| **Buenas Noches** (n=20) | 18.45 ± 1.23 | 12.35 ± 1.87 | 33 % | 7.15 ± 0.37 | 6.80 ± 0.41 | 5% |
| **Buenas Noches Total** (n=20) | 18 ± 1.62 | 8.45 ± 1.54 | 53% | 7.30 ± 0.57 | 5.55 ± 0.76 | 24% |

| | | | | | | |
|---|---|---|---|---|---|---|
| *ISI reduction or ISI decline rate = ((pre ISI score - post ISI score)/pre-ISI score) x 100%. **ESS reduction or ESS decline rate = ((pre EES score - post ESS score)/pre-ESS score) x 100%. | | | | | | |

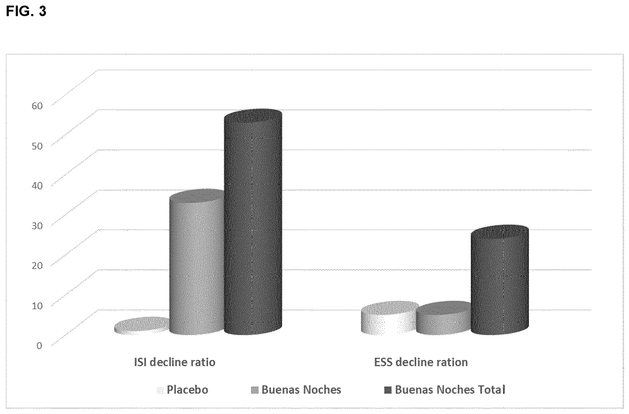

Both the Insomnia Severity Index and (ISI) and the level of daytime sleepiness (ESS score) were improved in *Buenas Noches Total* group, with an ISI and ESS decline rate of 53% and 24%, respectively (FIG. 3). No significant effect is observed in placebo group. *Buenas Noches* provided an ISI decline rate of 33% and no significant ESS decline rate.

Global assessment of the efficacy, tolerability and acceptance of the *Buenas Noches* and *Buenas Noches Total* treatment, showed:
a) *Buenas Noches*: Good or regular efficacy of 85% and 15% of patients, respectively; No patient rated the efficacy as bad.
   *Buenas Noches Total*: Good or regular efficacy of 95% and 5% of patients, respectively; No patient rated the efficacy as bad.
   *Placebo*: 20% of patients rated the efficacy as bad; Good or regular efficacy of 75% and 5% of patients, respectively.
b) *Buenas Noches*: Tolerability and acceptability was good in 95% of the cases, and regular in 5%. *Buenas Noches Total*: Tolerability and acceptability was good in 100% of the cases.
   *Placebo*: Tolerability and acceptability was good in 65% and 85% of patients, respectively.
c) 95% and 100% of the patients in the group treated with *Buenas Noches* or *Buenas Noches Total,* respectively, would repeat the treatment, compared to 80% of the placebo group.

Regarding safety, no adverse effects were observed in either treatment (*Buenas Noches* or *Buenas Noches Total*) or placebo groups.

### 1.3 Conclusions

The treatment with *Buenas Noches Total* in patients with primary insomnia demonstrated a high efficacy for the reduction of ISI and the level of ESS score, in this prospective, interventional, randomized, placebo-controlled and double-blind clinical investigation.

*Buenas Noches Total,* with an ISI decline rate of 53%, is significantly more effective than *Buenas Noches* (ISI decline rate of 33%), and also reduces the daytime sleepiness, with an ESS decline ratio of 24%.

### EXAMPLE 2: Efficacy and safety of Buenas Noches and Buenas Noches Total products on night-time sleep and daytime symptoms in patients with primary insomnia. Objective sleep quality evaluation by Kronohealth monitoring

### 2.1 Materials and methods

### 2.1.1 General information

The insomnia disorder was also diagnosed according to DSM-5 criteria.

Subjects, study design and conduct were the same as those detailed in EXAMPLE 1.1.1. Compositions were those detailed in EXAMPLE 1.1.2 and Table 1.

Eligibility criteria was the same as detailed in EXAMPLE 1.1.3.

Statistical analysis was performed as detailed in EXAMPLE 1.1.5.

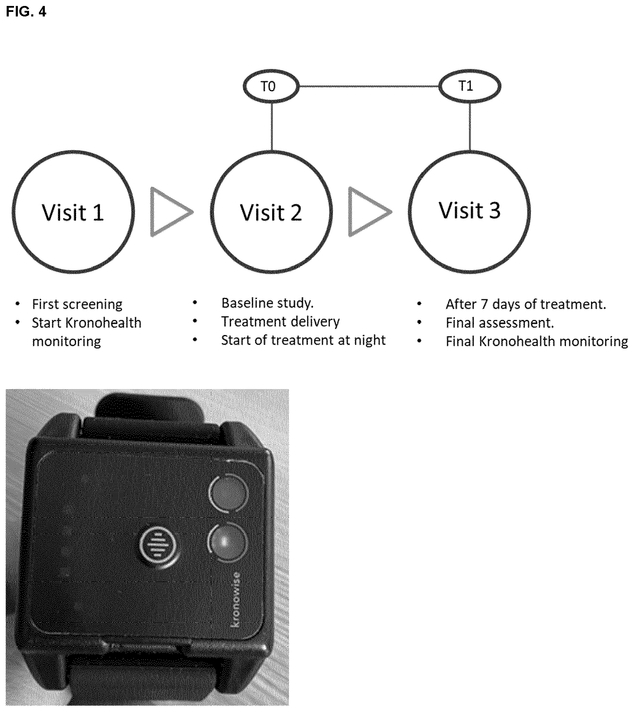

In this case, the duration of the study was 14 days per patient, with an initial period of sleep monitoring followed by the 7 days of treatment as described in EXAMPLE 1.1.1. There was a total of 3 visits. In the first visit (visit one), a device for the Ambulatory Monitoring of Sleep (Kronohealth monitoring, FIG. 4) was provided for all subjects, who wore this device every day at all times until the end of the study. In the second visit, either *Buenas Noches*, *Buenas Noches Total* or a placebo was provided to the patients (visit two, T0 as a mean of the results obtained in the previous week). The third visit was at the end of treatment (day 7 of the 1-week of treatment) (visit three, T1), when the bottles of the administered products (treatments and placebo) were returned to the principal investigator to assess compliance with the posology described in the protocol (see schedule in FIG. 4).

### 2.1.2 Primary and secondary endpoints

The primary endpoint of the study was to assess the clinical efficacy of *Buenas Noches* and *Buenas Noches Total* through the Kronohealth device (Ambulatory Monitoring of Sleep from the Chronobiology laboratory of the University of Murcia), recording these dependent variables:
- Start sleep (h:min): when the subject falls asleep.
- Stop sleep (h:min): when the subject wakes up (for the last time before getting up).
- Sleep onset latency (min): the period between bedtime and sleep start.
- Total sleep time (min): total time in bed, without sleep latency, awakenings or microawakenings.
- WASO (wake after sleep onset) (min): wake time (awakenings + microawakenings).
- Actual sleep time (min): total sleep period minus WASO.
- Sleep efficiency (%): sleep duration expressed as a percentage of time in bed.
- Bedtime: total time in bed.

T0 corresponds to the mean of data from previous 7 days without treatment.

The secondary endpoints were the evaluation of the satisfaction of the treatment, through questionnaires to the participants.

The clinical safety was evaluated through the registration of adverse effects in patients and the identification of those that are device-related adverse effects.

### 2.2 Results

Again, there were no differences in the sex ratio of the volunteers assigned to each study group (Placebo group: 50% men, 50% women; *Buenas Noches* group: 50% men, 50% women; *Buenas Noches Total* group: 50% men, 50% women; test of Pearson's chi-square: P = 1.000).

As detailed in Table 2, there were also no differences at the start of the study (T0) between volunteers included in the Placebo group, the *Buenas Noches* group and the *Buenas Noches Total* group in age (Kruskal-Wallis H Test: P = 0.995), height (Test Kruskal-Wallis H: P = 0.584), weight (Kruskal-Wallis H Test: P = 0.993), and BMI (Kruskal-Wallis H Test: P = 0.821).

All participants completed the entire study protocol.

Data obtained for different variables at baseline from the Kronohealth devices are included in Table 4, for the different treatments. For all variables recorded in the monitoring device, there were no differences at the start time of the study (T0) between participants included in the different groups (placebo, *Buenas Noches* and *Buenas Noches Total* groups).

**Table 4. Baseline data (T0) for different variables recorded from Kronohealth devices (mean ± SD).**

| **Variable** | **Placebo** | ***Buenas Noches*** | ***Buenas Noches Total*** |
|---|---|---|---|
| **Sleep latency (min)** | 31.5 ± 11.13 | 33.70 ± 11.70 | 33.90 ± 11.80 |
| **Total sleep time (min)** | 344.05 ±17.01 | 345.65 ± 10.56 | 345 ± 14.91 |
| **WASO (min)** | 21.00 ± 5.61 | 21.20 ± 6.16 | 21.80 ± 6.16 |
| **Actual sleep time (min)** | 323.05 ±18.99 | 324.45 ± 12.31 | 323.25 ± 13.08 |
| **Bedtime (min)** | 396.70 ± 18.99 | 401.90 ± 14.39 | 399.10 ± 14.68 |
| **Sleep efficiency (%)** | 81.56 ± 2.55 | 80.66 ± 2.68 | 81.02 ± 2.40 |

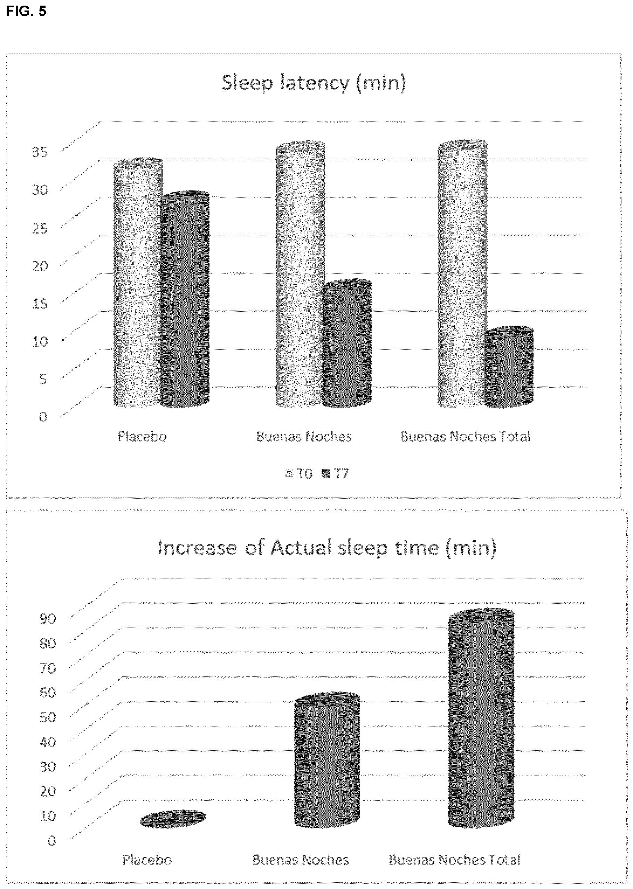

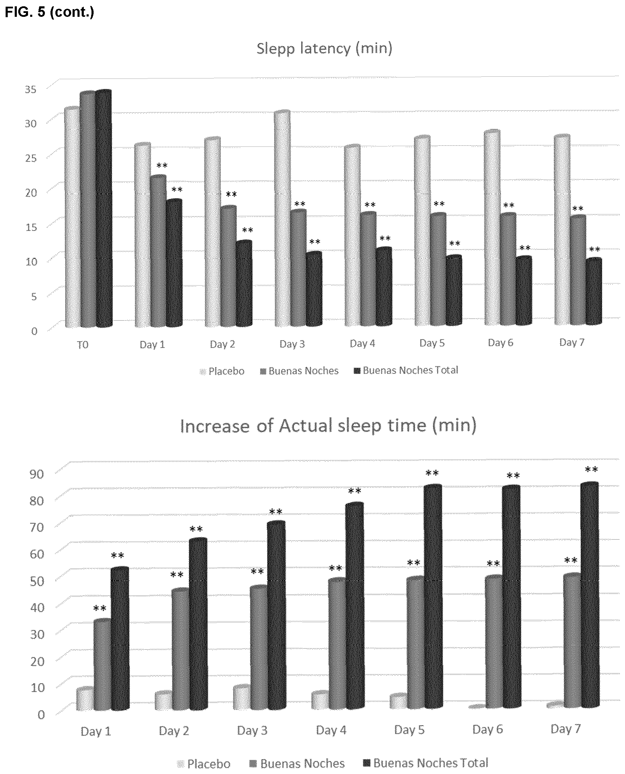

Table 5 and FIG. 5 show the evolution of the main variables (sleep latency and actual sleep time) from the start (T0) to the end of the study (T1) (during the week of treatment) measured by Kronohealth devices for the different treatments.

Table 5. Results (mean ± SD) obtained for the sleep latency and actual sleep time, during 7 days of treatment.

| **Variable** | | **Placebo** | ***Buenas Noches*** | ***Buenas Noches Total*** |
|---|---|---|---|---|
| **Sleep latency (min)** | T0 | 31.5 ± 11.13 | 33.70 ± 11.70 | 33.90 ± 11.80 |
| | Day 1 | 26.25 ± 9.39 | 21.50 ± 6.36 | 18.10 ± 6.17 |
| | Day 2 | 27.00 ± 8.50 | 17.10 ± 3.13 | 12.05 ± 3.85 |
| | Day 3 | 30.80 ± 8.17 | 16.50 ± 3.76 | 10.35 ± 3.18 |
| | Day 4 | 25.80 ± 7.71 | 16.10 ± 3.76 | 10.95 ± 2.35 |
| | Day 5 | 27.05 ± 6.09 | 15.90 ± 3.23 | 9.75 ± 2.10 |
| | Day 6 | 27.80 ± 5.65 | 15.85 ± 2.81 | 9.55 ± 2.01 |
| | Day 7 | 27.10 ± 4.48 | 15.46 ± 2.37 | 9.25 ± 1.86 |
| **Actual sleep time (min) (Total sleep time** - **WASO)** | T0 | 323.05 ±18.99 | 324.45 ± 12.31 | 323.25 ± 13.08 |
| | Day 1 | 330.55 ± 15.67 | 357.00 ± 11.33 | 375.15 ± 13.33 |
| | Day 2 | 328.85 ± 14.67 | 368.35 ± 10.96 | 385.90 ± 12.88 |
| | Day 3 | 331.05 ± 13.06 | 369.30 ± 9.47 | 392.15 ± 14.83 |
| | Day 4 | 328.65 ± 10.66 | 371.70 ± 10.75 | 398.75 ± 11.96 |
| | Day 5 | 327.55 ± 9.63 | 372.05 ± 9.20 | 405.30 ± 9.53 |
| | Day 6 | 323.10 ± 18.92 | 372.30 ± 10.37 | 404.90 ± 10.22 |
| | Day 7 | 324.00 ± 8.38 | 372.90 ± 8.87 | 405.95 ± 8.32 |

Sleep latency, i.e., the duration from lights out (lights turned off) while the patient attempts to sleep until they fall asleep, is perhaps one of the most important parameters in a sleep study.

After one day of treatment, *Buenas Noches* and *Buenas Noches Total* reduced the sleep latency in 13 minutes (reduction of 38%, sleep latency of 21 minutes *versus* 34 minutes in initial time) and 16 minutes (reduction of 47%, sleep latency of 18 minutes *versus* 34 minutes in initial time), respectively (p<0.001).

After 7 days of treatment, *Buenas Noches* and *Buenas Noches Total* reduced the sleep latency in 19 minutes (reduction of 56% in comparison with baseline, sleep latency of 15 minutes *versus* 34 minutes in initial time) and 25 minutes (reduction of 74% in comparison with baseline, sleep latency of 9 minutes *versus* 34 minutes in initial time), respectively (p<0.001). However, subjects from placebo group only reduced the sleep latency by 4 minutes (sleep latency of 27 minutes *versus* 31 minutes in initial time).

Wake after sleep onset (WASO) is also an important reported parameter, which refers to the periods of wakefulness occurring after the defined sleep onset. This parameter measures wakefulness excluding wakefulness occurring before sleep onset. The WASO time better reflects the fragmentation of sleep and allows to obtain the calculation of the actual sleep time (total sleep time - WASO).

Participants of the placebo group did not increase actual sleep time (5 hours and 40 minutes). However, after 7 days of treatment, *Buenas Noches* and *Buenas Noches Total* increased the actual sleep time by 49 minutes (14.9% increase) and 83 minutes (25.6% increase), respectively, compared to baseline. This increase in sleep time is observed from day one in 33 minutes and 52 minutes, respectively; reaching an increase of 44 minutes and 63 minutes after the second day of treatment.

Sleep efficiency is another important parameter that refers to the percentage of total time in bed. Table 6 shows the observed changes (T1 versus T0) in Sleep efficiency (%) and Bedtime, for the different treatments.

**Table 6. Results (mean ± SD) obtained for the bedtime and sleep efficiency, after 7 days of treatment.**

| **Variable** | | **Placebo** | ***Buenas Noches*** | ***Buenas Noches Total*** |
|---|---|---|---|---|
| **Bedtime (min)** | T0 | 396.70 ± 18.99 | 401.90 ± 14.39 | 399.10 ± 14.68 |
| | Day 7 | 396.05 ± 8.38 | 372.90 ± 8.87 | 405.95 ± 8.32 |
| **Sleep efficiency (%)** | T0 | 81.56 ± 2.55 | 80.66 ± 2.68 | 81.02 ± 2.40 |
| | Day 7 | 81.85 ± 1.91 | 89.94 ± 0.86 | 93.20 ± 1.12 |

The sleep efficiency for patients included in the *Buenas Noches* group was of 90% (*versus* 81% at baseline), and 93% in the *Buenas Noches Total* group (*versus* 81% at baseline).

Global assessment of the efficacy, tolerability and acceptance of the *Buenas Noches* and *Buenas Noches Total* treatment, showed same results as detailed in EXAMPLE 1.2.

Regarding safety, no adverse effects were observed in either treatment (*Buenas Noches* or *Buenas Noches Total*) or placebo groups.

### 2.3 Conclusions

The main difference of *Buenas Noches Total* versus *Buenas Noches* is the addition of a small quantity of glycine. Both products showed an improvement in different parameters related to primary insomnia, but *Buenas Noches Total* group presented a more significant efficacy.

After 7 days of treatment, *Buenas Noches* and *Buenas Noches Total* reduced sleep latency in 19 minutes (reduction of 56% in comparison with baseline, sleep latency of 15 minutes *versus* 34 minutes in initial time) and 25 minutes (reduction of 74% in comparison with baseline, sleep latency of 9 minutes *versus* 34 minutes in initial time), respectively (p<0.001). However, subjects from placebo group only reduced the sleep latency in 4 minutes (sleep latency of 27 minutes *versus* 31 minutes in initial time).

After 7 days of treatment, *Buenas Noches* and *Buenas Noches Total* increased the actual sleep time for 49 minutes and 83 minutes, respectively, compared to baseline. This sleep time increase is observed from day one in 33 minutes and 52 minutes, respectively; reaching an increase of 44 minutes and 63 minutes after the second day of treatment.

## Claims

1. A composition comprising:
glycine in a range of between 25 and 150 mg;
gamma-aminobutyric acid in a range of between 50 and 250 mg;
at least one vegetal extract selected from the group consisting of *Valerian* root dry extract, *Passiflora* dry extract, and *Californian poppy* dry extract; and
melatonin in an amount of less than 2 mg.

2. The composition according to claim 1, wherein the composition further comprises at least one vitamin in a range of between 15 and 20 mg.

3. The composition according to claim 2, wherein the vitamin is a vitamin B.

4. The composition according to claim 3, wherein vitamin B is selected from the group consisting of vitamin B3, vitamin B5 and vitamin B6.

5. The composition according to any of claims 1-4, wherein the composition comprises *Valerian* root dry extract, *Passiflora* dry extract, and *Californian poppy* dry extract.

6. The composition according to any of claims 1-5, wherein the composition comprises glycine in a range of between 25 and 150 mg; gamma-aminobutyric acid in a range of between 50 and 250 mg; *Valerian* root dry extract in a range of between 80 and 120 mg; *Passiflora* dry extract in a range of between 60 and 90 mg; *Californian poppy dry* extract in a range of between 15 and 30 mg; melatonin in an amount of less than 2 mg; vitamin B3 in a range of between 10 and 20 mg; and vitamin B6 in a range of between 1 and 1.8 mg.

7. The composition according to claim 6, wherein the composition comprises 50 mg of glycine, 100 mg of gamma-aminobutyric acid, 100 mg of *Valerian* root dry extract, 75 mg of *Passiflora* dry extract, 25 mg of *Californian poppy* dry extract, 1.85 mg of melatonin, 16 mg of vitamin B3, and 1.4 mg of vitamin B6.

8. The composition according to any of claims 1-7, wherein the composition is in the form of a nutraceutical product, a food supplement, a medical food, a food product a pharmaceutical product, or a veterinary product, and wherein the composition comprises appropriate amounts of acceptable excipients.

9. The composition according to any of claims 1-8, wherein the composition is in a form selected from the group consisting of a spray, a gel, a tablet and a gummy.

10. The composition according to claim 9, wherein the tablet is a bilayer or tri-layer tablet that comprises an external layer and an internal layer, the external layer comprising melatonin.

11. The composition according to any of claims 1-10, wherein the composition has a total weight of between 500 and 700 mg.

12. The composition according to any of claims 1-11, for use in treating a sleep disorder.

13. The composition for use according to claim 12, wherein the sleep disorder is insomnia disorder.

14. The composition for use according to any of claims 12-13, wherein the composition:
i) reduces the insomnia severity measured by ISI score;
ii) reduces the sleepiness measured by ESS score;
iii) reduces the sleep latency;
iv) increases the actual sleep time; and/or
vi) improves the sleep efficiency.

15. The composition for use according to claim 14, wherein the composition:
i) reduces the insomnia severity by reducing the ISI score in about 50%;
ii) reduces the sleepiness by reducing the ESS score in about 20%;
iii) reduces the sleep latency in about 70%;
iv) increases the actual sleep time in about 25%; and
v) improves the sleep efficiency in about 14%,
after 7 days of treatment compared to baseline.
